# EUROPEAN PATENT APPLICATION

(11) **EP 4 249 510 A1**
(43) Date of publication of application: **27.09.2023**
(21) Application number: 22305338.0
(22) Date of filing: 22.03.2022
(51) Int. Cl.: C07K 16/28, C07K 14/705

(54) **NOVEL FORMATS OF ANTI-CD28/SPD-1 FUSION CONSTRUCTS**

(71) Applicant: Stealth IO, 13009 Marseille (FR); Université d'Aix-Marseille, 13007 Marseille (FR); Centre national de la recherche scientifique, 75016 Paris (FR); INSTITUT NATIONAL DE LA SANTE ET DE LA RECHERCHE MEDICALE - INSERM, 75013 Paris (FR); Institut Jean Paoli & Irène Calmettes, 13009 Marseille (FR)
(72) Inventor: OLIVE, Daniel, 13009 MARSEILLE (FR); ELSON, Greg, CH-1206 GENEVE (CH)
(74) Representative: Lavoix

(57) **Abstract**

The invention relates to a bispecific protein complex of a defined structure, said complex being able to bind to CD28 as well as to PD-L1 and/or PD-L2.

It also relates to the use of said bispecific protein complex as a medicament, or for treating a cancer.

## Description

### FIELD OF THE INVENTION

The present invention concerns novel fusion constructs targeted against both CD28 and PD-1 ligands.

### BACKGROUND OF THE INVENTION

Over the past decade, a growing understanding of the molecular mechanisms that allow cancer cells to evade detection by the immune system has led to the emergence of immunotherapies as a new pillar of cancer treatment. Both innate and adaptive immune responses are governed by the balance of positive and negative signals mediated by immune checkpoints. These checkpoint molecules fine-tune the immune response, with inhibitory pathways (such as programmed cell death protein 1 (PD-1) or cytotoxic T lymphocyte-associated protein 4 (CTLA4)) that attenuate T cell activation, and costimulatory pathways (such as CD28 or OX40) that accelerate T cell activation. The binding of PD-1 to its ligands PD-L1 and PD-L2 suppresses the activation and function of T cells to downregulate the immune response.

Clinical efficacy has now been observed with checkpoint inhibitors such as anti-CTLA-4 or anti-PD-1 monoclonal antibodies (Ribas, A. & Wolchok, J. D. Cancer immunotherapy using checkpoint blockade. Science 359, 1350-1355 (2018)). However, most patients do not respond to immunotherapy or inevitably develop resistance to treatment after a period of treatment.

Further enhancements are thus needed for a more effective treatment.

One approach to optimize the efficacy of immunotherapy is to use bispecific or multispecific antibodies, called T cell engagers, that recognize tumor targets and simultaneously interact with T cells, redirecting them to lyse tumor targets (Goebeler, Maria-Elisabeth; Bargou, Ralf C. T cell-engaging therapies - BiTEs and beyond. Nature Reviews Clinical Oncology. 17(7):418-434 (2020)). As an example of said therapeutic strategy, the dual specificity of blinatumomab to CD19 and CD3 led to improved outcomes in the treatment of B cell lymphoma (Przepiorka, D. et al. FDA approval: blinatumomab. Clin. Cancer Res. 21, 4035-4039 (2015)).

However said antibodies do not present both a tumoral specificity and an immunomodulatory function.

Two signals are required to stimulate T cells for optimal effector function and sustained proliferation. CD3 stimulation represents the « primary signal » required for T cell proliferation. Stimulation of the primary signal pathway alone, in the absence of a second signal, promotes activation-induced cell death that may limit the durability of the anti-tumor response. Engagement of a second surface membrane protein, CD28, stimulates an alternative signal transduction pathway and inhibits programmed cell death, to promote the survival of the activated T cells.

However, CD28 ligands (CD80 and CD86) are downregulated on most of the tumor cells. In addition, PD-1 suppresses T cell function primarily by inactivating CD28 costimulation.

A soluble PD-1 protein (sPD-1), through binding to its ligands PD-L1/L2 on tumor cells, could prevent the PD-1 on T cells from being engaged, thereby avoiding T cell inhibition. PD-L1 has also been reported to bind to CD80, resulting in T cell inhibition in an indirect manner by preventing CD80:CD28 interaction (Butte MJ, Keir ME, Phamduy TB, Sharpe AH, Freeman GJ. Programmed death-1 ligand 1 interacts specifically with the B7-1 costimulatory molecule to inhibit T cell responses. Immunity 27(1):111-22 (2007)). Hence, in addition to blocking PD-1/PD-L1 and PD-1/PD-L2 interactions, sPD-1 may also interrupt PD-L1/CD80 interaction by binding to PD-L1.

Thus, it is clear that there is a need for improving the currently available therapies for the treatment of cancers.

### SUMMARY OF THE INVENTION

The inventors have synthetized novel constructs which target and bind to both CD28 and PD-1 ligands (PD-L1 and/or PD-L2). These constructs comprise antibody fusion proteins, and have been designed (i) to bind to PD-L1 and/or PD-L2 on tumor cells, thus blocking PD-1 inhibitory signaling, and simultaneoulsy (ii) to bind to CD28 on T cells, thus recruiting T cells and resulting in T cell activation and proliferation.

Accordingly, the present invention relates to a bispecific protein complex having the formula A:B-C-X:(D)ₘ-C'-Y wherein:
A is chosen from VL-(CL)ₙ, VL-(CH1)ₙ and VH, wherein
   VL is a light chain variable region of an antibody directed against CD28,
   CL is a light chain constant region of an antibody,
   CH1 is the first domain of a heavy chain constant region of an antibody, and
   VH is the heavy chain variable region of an antibody directed against CD28 which forms with VL a binding site to CD28, and
n is an integer which is 0 or 1,B-C-X is a first fusion protein, wherein :
   B is chosen from VH-(CH1)ₙ, VH-(CL)ₙ and VL, wherein VH, CH1, CL, VL and n are as defined for A,
   C is the hinge-CH2-CH3 domain of an antibody,
   X is a soluble PD-1 protein or one of its fragments, and
   the C-terminal end of the CH3 domain of C is linked to the N-terminal end of X ;
with the proviso than when A is VH, then B is VL, and
with the proviso than when n is 1, then A is VL-CL and B is VH-CH1, or A is VL-CH1 and B is VH-CL,
(D)ₘ-C'-Y is a second fusion protein, wherein :
   D is a protein or a protein dimer,
   m is an integer which is 0 or 1,
   C' is a amino acid sequence which shows at least 80% identity, preferably at least 85% identity, preferably at least 90% identity, preferably at least 95% identity with C ;
   Y is a soluble PD-1 protein or one of its fragments, and Y is identical to or different from X, and
   the C-terminal end of the CH3 domain of C' is linked to the N-terminal end of Y ;
: is a binding interaction respectively between A and B, and between C of the first fusion protein and C' of the second fusion protein.

It also relates to the use of the bispecific protein complex of the invention as a medicament.

It also relates to the use of the bispecific protein complex of the invention for treating a cancer.

### DETAILED DESCRIPTION OF THE INVENTION

The anti-CD28/sPD-1 constructs have been rationally designed to bind to PD-L1 and PD-L2 on tumor cells, thus blocking PD-1 inhibitory signaling, and simultaneoulsy bind to the costimulatory CD28 receptor, thus recruiting T cells and resulting in T cell activation and proliferation. The constructs of the invention are designed in either monovalent or bivalent format for binding CD28, and comprise either a wild type or mutated sPD-1 protein fused to the C-terminus of the Fc via a conventional glycine-serine linker (GGGGS)_{P} with p being an integer from 1 to 5, preferably 3.

The present invention thus relates to a bispecific protein complex having the formula A:B-C-X:(D)ₘ-C'-Y wherein:
A is chosen from VL-(CL)ₙ, VL-(CH1)ₙ and VH, wherein
   VL is a light chain variable region of an antibody directed against CD28,
   CL is a light chain constant region of an antibody,
   CH1 is the first domain of a heavy chain constant region of an antibody, and
   VH is the heavy chain variable region of an antibody directed against CD28 which forms with VL a binding site to CD28, and
n is an integer which is 0 or 1,B-C-X is a first fusion protein, wherein :
   B is chosen from VH-(CH1)ₙ, VH-(CL)ₙ and VL, wherein VH, CH1, CL, VL and n are as defined for A,
   C is the hinge-CH2-CH3 domain of an antibody,
   X is a soluble PD-1 protein or one of its fragments, and
   the C-terminal end of the CH3 domain of C is linked to the N-terminal end of X ;
with the proviso than when A is VH, then B is VL, and
with the proviso than when n is 1, then A is VL-CL and B is VH-CH1, or A is VL-CH1 and B is VH-CL,
(D)ₘ-C'-Y is a second fusion protein, wherein :
   D is a protein or a protein dimer,
   m is an integer which is 0 or 1,
   C' is a amino acid sequence which shows at least 80% identity, preferably at least 85% identity, preferably at least 90% identity, preferably at least 95% identity with C ;
   Y is a soluble PD-1 protein or one of its fragments, and Y is identical to or different from X, and
   the C-terminal end of the CH3 domain of C' is linked to the N-terminal end of Y ;
   : is a binding interaction respectively between A and B, and between C of the first fusion protein and C' of the second fusion protein.

Such bispecific protein complexes of the invention may present various formats.

First, they are always bispecific. "Bispecific" as employed herein refers to the ability to bind two different target antigens, which are CD28 and a ligand of PD-1 (PD-L1 and/or PD-L2).

Second, they may be monovalent or bivalent. By "monovalent" according to the invention, it is meant that the bispecific protein complex comprises only one binding site for CD28 (one single binding site for CD28). By "bivalent" according to the invention, it is meant that the bispecific protein complex comprises two binding sites for CD28.

In any case, the bispecific protein complexes of the invention are always bivalent for binding to PD-L1 and/or PD-L2, as they comprise two binding sites to PD-L1 and/or PD-L2, namely domains X and Y.

The bispecific protein complex of the invention comprises different antibody fragments, which are defined below, in a given order.

### Definitions

### Antibody and antibody fragments

An antibody (or "immunoglobulin") consists of a glycoprotein comprising at least two heavy (H) chains and two light (L) chains interconnected by disulfide bonds. Each heavy chain comprises a heavy chain variable region (or domain) (abbreviated herein as VH) and a heavy chain constant region (hereafter CH). Heavy chains are classified as gamma, mu, alpha, delta or epsilon, and define the antibody's isotype as IgG, IgM, IgA, IgD and IgE, respectively. The heavy chain constant region of the immunoglobulin IgG, IgD, and IgA (g, d and a chains respectively) comprises three domains (CH1, CH2, and CH3) and a hinge region for added flexibility, and the heavy chain constant region of the immunoglobulin IgM and IgE contains 4 domains (CH1, CH2, CH3 and CH4).

By « antibody directed against CD28 », it is meant an antibody that binds CD28 via at least one binding site, or two binding sites. Preferably, said antibody is the monoclonal antibody 9.3, known from Hansen JA et al, Immunogenetics, 1980, or its humanized version.

The antibody may also comprise, in an embodiment, a VHH region instead of VH. The term "VHH" refers to the single heavy chain variable domain of antibodies of the type that can be found in Camelid mammals which are naturally devoid of light chains; synthetic VHH can be construed accordingly. Such VHH are also called "single domain antibody" (sdAb). Preferably, VHH can particularly be llama VHH. When the antibody comprises a VHH region instead of VH, then it is devoid of light chains.

Preferably, the bispecific protein complex of the invention comprises fragments of the IgG isotype, i.e. the heavy chain is of the gamma (y) type. IgG antibodies are classified in four distinct subtypes, named IgG1, IgG2, IgG3 and IgG4 in order of their abundance in serum (IgG1 being the most abundant). The structure of the hinge regions in the g chain gives each of these subtypes its unique biological profile (even though there is about 95% similarity between their Fc regions, the structure of the hinge regions is relatively different). The bispecific protein complex of the invention can comprise fragments of the IgG1, IgG2, IgG3 or IgG4 subtype, preferably of the IgG1 subtype or of the IgG2 subtype. More preferably, the bispecific protein complex of the invention comprises fragments of the IgG1 subtype.

Each light chain of an antibody comprises a light chain variable region (abbreviated herein as VL) and a light chain constant region comprising only one domain, CL. There are two types of light chain in mammals: the kappa (K) chain, encoded by the immunoglobulin kappa locus on chromosome 2, and the lambda (A) chain, encoded by the immunoglobulin lambda locus on chromosome 22. Preferably, the bispecific protein complex of the invention has a kappa light chain.

According to the invention, the VL and the VH of the bispecific protein complex belong to an antibody directed against CD28, preferably the same single one. The VL and VH form a binding site to CD28.

According to the invention, preferably, the CDR sequences of the VL and the VH of the bispecific protein complex belong to an antibody directed against CD28.

Preferably, the VL, VH, CH1 and hinge-CH2-CH3 domain of the bispecific protein complex of the invention belong to an antibody directed against CD28, preferably belong to one single antibody directed against CD28. Preferably, the VL, VH, CH1 and hinge-CH2-CH3 domain of the bispecific protein complex of the invention belong to monoclonal antibody 9.3.

More preferably, the anti-CD28 antibody light and heavy chains comprise the following first combination of 6 CDRs :
as in Kabat:
H-CDR1: DYGVH (SEQ ID NO: 11)
H-CDR2: VIWAGGGTNYNSALMS (SEQ ID NO: 12)
H-CDR3: DKGYSYYYSMDY (SEQ ID NO: 13),
and
L-CDR1: RASESVEYYVTSLMQ (SEQ ID NO: 14)
L-CDR2: AASNVES (SEQ ID NO: 15)
L-CDR3: QQSRKVPYT (SEQ ID NO: 16).

Said first combination of CDRs is as follows in IMGT :
H-CDR1: GFSLSDYG (SEQ ID NO: 17)
H-CDR2: IWAGGGT (SEQ ID NO: 18)
H-CDR3: ARDKGYSYYYSMDY (SEQ ID NO: 19),
and
L-CDR1: ESVEYYVTSL (SEQ ID NO: 20)
L-CDR2: AA (SEQ ID NO: 21)
L-CDR3: QQSRKVPYT (SEQ ID NO: 16).

Said above first combination corresponds to murine sequences of clone 9.3.

Alternatively, more preferably, the anti-CD28 antibody light and heavy chains comprise the following second combination of 6 CDRs :
as in Kabat:
H-CDR1: DYGVH (SEQ ID NO: 11)
H-CDR2: AIWAGGGTNYASSVMG (SEQ ID NO: 22)
H-CDR3: DKGYSYYYSMDY (SEQ ID NO: 13),
and
L-CDR1: RASESVEYYVTSLMA (SEQ ID NO: 23)
L-CDR2: AASNVES (SEQ ID NO: 15)
L-CDR3: QQSRKVPYT (SEQ ID NO: 16).

Said second combination of CDRs is as follows in IMGT :
H-CDR1: GFTFSDYG (SEQ ID NO: 24)
H-CDR2: IWAGGGT (SEQ ID NO: 18)
H-CDR3: ARDKGYSYYYSMDY (SEQ ID NO: 19),
and
L-CDR1: ESVEYYVTSL (SEQ ID NO: 20)
L-CDR2: AA (SEQ ID NO: 21)
L-CDR3: QQSRKVPYT (SEQ ID NO: 16).

Said above second combination corresponds to humanized sequences of clone 9.3.

Alternatively, preferably, the anti-CD28 antibody light and heavy chains comprise the sequence SEQ ID NO:25 as VL domain, and the sequence SEQ ID NO:26 as VH domain.

Alternatively, the anti-CD28 antibody light and heavy chains comprise the following third combination of 6 CDRs, as in Kabat:
H-CDR1: GGSISSYY (SEQ ID NO:35)
H-CDR2: IYYSGIT (SEQ ID NO:36)
H-CDR3: ARWGVRRDYYYYGMDV (SEQ ID NO:37),
and
L-CDR1: QSVSSSY (SEQ ID NO:38)
L-CDR2: GAS (SEQ ID NO:39)
L-CDR3: QQYGSSPWT (SEQ ID NO:40).

Alternatively, preferably, the anti-CD28 antibody light and heavy chains comprise the sequence SEQ ID NO:41 as VL domain, and the sequence SEQ ID NO:42 as VH domain.

Alternatively, the anti-CD28 antibody light and heavy chains comprise the following fourth combination of 6 CDRs, as in Kabat:
H-CDR1 chosen from GFTFSSYG (SEQ ID NO:43) and GFTFSRNN (SEQ ID NO:44)
H-CDR2 chosen from ISYAGNNK (SEQ ID NO:45) and ISSNGGRT (SEQ ID NO:46)
H-CDR3 chosen from AKDSYYDFLTDPDVLDI (SEQ ID NO:47) and TRDDELLSFDY (SEQ
ID NO:48),
and
L-CDR1: QSISSY (SEQ ID NO:49)
L-CDR2: AAS (SEQ ID NO:50)
L-CDR3: QQSYSTPPIT (SEQ ID NO:51).

Alternatively, preferably, the anti-CD28 antibody light and heavy chains comprise the sequence SEQ ID NO:52 as VH domain, and the sequence SEQ ID NO:53 as VL domain.

Alternatively, preferably, the anti-CD28 antibody light and heavy chains comprise the sequence SEQ ID NO:54 as VH domain, and the sequence SEQ ID NO:55 as VL domain.

Alternatively, the anti-CD28 antibody light and heavy chains comprise the following fifth combination of 6 CDRs, as in Kabat:
H-CDR1: GFTFSRNN (SEQ ID NO:44)
H-CDR2: ISSNGGRT (SEQ ID NO:46)
H-CDR3: TRDDELLSFDY (SEQ ID NO:48),
and
L-CDR1: QSISSY (SEQ ID NO:49)
L-CDR2: AAS (SEQ ID NO:50)
L-CDR3: QQSYSTPPIT (SEQ ID NO:51).

Alternatively, preferably, the anti-CD28 antibody light and heavy chains comprise the sequence SEQ ID NO:55 as VL domain, and the sequence SEQ ID NO:56 as VH domain.

Alternatively, the anti-CD28 antibody light and heavy chains comprise the following sixth combination of 6 CDRs, as in Kabat:
H-CDR1: GYTFTSYY (SEQ ID NO:57)
H-CDR2: IYPGNVNT (SEQ ID NO:58)
H-CDR3: TRSHYGLDWNFDV (SEQ ID NO:59),
and
L-CDR1: QNIYVW (SEQ ID NO:60)
L-CDR2: KAS (SEQ ID NO:61)
L-CDR3: QQGQTYPY (SEQ ID NO:62).

Alternatively, preferably, the anti-CD28 antibody light and heavy chains comprise the sequence SEQ ID NO:63 as VL domain, and the sequence SEQ ID NO:64 as VH domain.

Alternatively, the anti-CD28 antibody light and heavy chains comprise the 6 CDRs of clone 1A7[CD28] H1L1 as described on figure 18 of WO2022/040482:
H-CDR1: SYAMS (SEQ ID NO:65)
H-CDR2: TISGSGDSTYYADSVKG (SEQ ID NO:66)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQSYSTPFT (SEQ ID NO:70),
or the 6 CDRs of clone 1A7[CD28] H1 L1.71:
H-CDR1: SYAMS (SEQ ID NO:65)
H-CDR2: TISGSGDSTYYADSVKG (SEQ ID NO:66)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQVYSTPFT (SEQ ID NO:71),
or the 6 CDRs of clone 1A7[CD28] H1.1 L1.71 as described on figure 21A of WO2022/040482 :
H-CDR1: SYYMS (SEQ ID NO:72)
H-CDR2: TISGSGDSTYYADSVKG (SEQ ID NO:66)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQVYSTPFT (SEQ ID NO:71),
or the 6 CDRs of clone 1A7[CD28] H1.14 L1:
H-CDR1: SYYMS (SEQ ID NO:72)
H-CDR2: TISESGDSTYYADSVKG (SEQ ID NO:73)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQSYSTPFT (SEQ ID NO:70),
or the 6 CDRs of clone 1A7[CD28] H1.14 L1.71 as described on figure 21B of WO2022/040482 :
H-CDR1: SYYMS (SEQ ID NO:72)
H-CDR2: TISESGDSTYYADSVKG (SEQ ID NO:73)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQVYSTPFT (SEQ ID NO:71).

The VH and VL regions can be further subdivided into regions of hypervariability, termed "Complementarity Determining Regions" (CDR), which are primarily responsible for binding an epitope of an antigen, and which are interspersed with regions that are more conserved, termed "Framework Regions" (FR). Each VH and VL is composed of three CDRs and four FRs, arranged from amino-terminus to carboxy-terminus in the following order: FR1, CDR1, FR2, CDR2, FR3, CDR3, FR4. The assignment of amino acid sequences to each domain is in accordance with well-known conventions (Chothia C, Lesk AM, Tramontano A, et al. Conformations of immunoglobulin hypervariable regions. Nature 1989; 342 : 877-83 ; and Chothia C, Lesk AM. Canonical structures for the hypervariable regions of immunoglobulins. J Mol Biol 1987; 196 : 901-17). The functional ability of an antibody to bind a particular antigen, here CD28, depends on the variable regions of each light/heavy chain pair and is largely determined by the CDRs. The variable region of the heavy chain differs in antibodies produced by different B cells, but is the same for all antibodies produced by a single B cell or B cell clone (or hybridoma). By contrast, the constant regions of the antibodies (Fc region) mediate the binding of the immunoglobulin to host tissues or factors, including various cells of the immune system (e.g. effector cells) and the first component (C1q) of the classical complement system. The binding of the Fc region to a Fc receptor leads to effector functions. Said effector functions include antibody-dependent cell-mediated cytotoxicity (or ADCC), antibody-dependent cellular phagocytosis (ADCP) and complement-dependent cytotoxicity (CDC).

ADCC refers to a form of cytotoxicity in which secreted Ig bound onto Fc receptors (FcRs) present on certain cytotoxic cells (e.g., Natural Killer (NK) cells, neutrophils, and macrophages) that enables these cytotoxic effector cells to bind specifically to an antigenbearing target cell and subsequently kill the target cell with cytotoxins. Ligand specific highaffinity IgG antibodies directed to the surface of target cells stimulate the cytotoxic cells and are absolutely required for such killing. Lysis of the target cell is extracellular, requires direct cell-to-cell contact, and does not involve complement. The ability of any particular antibody to mediate lysis of the target cell by ADCC can be assayed by any method known in the art.

ADCP refers to the process by which antibody-coated cells are internalized, either in whole or in part, by phagocytic immune cells (e.g., macrophages, neutrophils and dendritic cells) that bind to an immunoglobulin Fc region.

CDC refers to the form of cytotoxicity in which the complement cascade is activated by the complement component C1q binding to antibody Fc.

The Fc region of the anti-CD28 antibody may be wild-type or mutated. According to an embodiment, the Fc region is mutated as compared to a parent Fc region. Then preferably it comprises at least one mutation that inhibits effector functions, preferably that inhibits (i.e. decreases) at least ADCC, ADCP or CDC. By « mutation », it is meant an amino acid insertion, an amino acid deletion or an amino acid substitution by a natural or an unnatural amino acid residue.

Said mutations that inhibit effector functions are well-known in the art. Preferably, the mutation is chosen from L234A, L235A, L234F, L235E, P331S, P329G, N297Q, D265A, N297A, N297Q, N297G and their combinations. More preferably, the mutation is chosen fromL234A/L235A, L234F/L235E/P331S L234A/L235A/P329G and L234F/L235E/D265A.

The reduced Fc effector function may also be reached by using IgG4 as the choice of isotype, preferably human IgG4 (hlgG4). Although not Fc-engineering per se, it has long been known that different hlgG isotypes convey varying levels of effector function which was later attributed to their differential binding to FcRs and C1q (Bindon et al., 1988). In particular, the hIgG4 isotype is known to have low affinity for all FcR, except FcRI. Preferably, when a Fc of hIgG4 is used, it comprises a substitution of the serine at position 228 to prevent F(ab) arm exchange.

The heavy chain constant domain CH3 may be a wild-type, or may be engineered by the knob-into-holes technology. Said technology allows the effective dimerization of heavy chains and facilitates heterodimerization. To reduce nonproductive assembly of molecules containing 2 VH or 2 VL modules, complementary knobs-into-holes mutations may be set into the heavy chains, preferably heavy chains of IgG. These mutations are well-konwn in the art, see for example Merchant, A.M., et al., Nat Biotechnol 16 (1998) 677-681 and Ridgway, J.B., et al., Protein Eng 9 (1996) 617-621. They force heterodimerization of differentheavy chains and consist of a T366W mutation in one heavy chain and T366S, L368A and Y407V mutations in the corresponding other chain.

The term "Fab fragment" as used herein refers to an antibody fragment comprising a light chain fragment comprising a variable domain (VL) and a constant domain (CL) of a light chain, and a variable domain (VH) and a first constant domain (CH1) of a heavy chain. Said Fab includes an antigen binding site of the intact antibody and thus retaining the ability to bind antigen, here CD28.

The Fab fragment may also be used in the form of a unique chain, in which VL-CL and VH-CH1 are fused by a linker.

The term « scFv » as used herein refers to a single-chain variable fragment (scFv). Said fragment is a fusion protein of the VH and VL regions, which are connected with a linker. The linker can connect the N-terminal end of the VH with the C-terminal end of the VL, or vice-versa. The scFv of the invention also binds CD28.

### PD-1 and its liaands

Membrane-bound PD-1 (full-length PD-1) is a type I transmembrane glycoprotein belonging to CD28 family of receptors gene. Human PD-1 is a 55 kDa glycoprotein of 288 amino acids, the sequence of which is available in Uniprot under acession number Q15116. It is composed of the signal sequence (23 amino acids) fused to N-terminal IgV like domain (147 amino acids), a transmembrane domain (21 amino acids), and a 97 amino-acid intracytoplasmic domain that contains two tyrosine-based signaling motifs. PD-1 lacks intracellular SH2 (Src Homology 2) or SH3 binding motifs unlike CD28 and CTLA-4. PD-1 is produced as monomer as it lacks the cysteine residue that is required for homodimerization (Khan et al, Front. Immunol., 19 November 2020, Soluble PD-1: Predictive, Prognostic, and Therapeutic Value for Cancer Immunotherapy).

The signal sequence fused to N-terminal IgV like domain is the extracellular fragment, or soluble form (sPD-1) ; it comprises 170 amino acids. The N-terminal IgV like domain is the mature soluble form and comprises 147 amino acids. However, some amino acids in Nor C-terminal may be deleted, and correspond to fragments, without any impact on the functionality of the molecule.

Thus, by « soluble PD-1 protein », it is meant a protein which comprises the N-terminal IgV like domain and optionally the signal sequence, but which does not comprise the transmembrane domain nor the intracytoplasmic domain.

Said sPD-1 may be wild-type, or comprises at least one mutation as compared to the wild-type ("mutated sPD-1"). By « mutation », it is meant an amino acid insertion, an amino acid deletion or an amino acid substitution by a natural or an unnatural amino acid residue.

Preferably, the human wild-type sPD-1 of the invention is SEQ ID NO :10.

Alternatively, preferably, the mutation of sPD-1 increases the affinity of PD-1 to PD-L1 and/or PD-L2 as compared to the wild-type protein. Preferably, the mutation is a substitution. Preferably the mutation is selected from G124S, K131Y, A132I, A132V, A132L, V64H, N66I, N66V, Y68H, M70E, M70I, N74G, T76P, K78T, S87G, S87W, C93A, N116S, L122V, A125V, S127V, S127A, K135M, A140V and their combinations, wherein the amino acid numbering is the one of human wild-type full-length PD-1 protein, and provided that when at least two mutations are present, they do not occur on the same position.

Alternatively, preferably, the mutation of sPD-1 improves the level of expression or eases its manufacturability as compared to the wild-type protein. Preferably, the mutation is a substitution. Preferably the mutation occurs at least on one of the positions N49, N58, N74 or N116, where the asparagine residue which is part of a N-glycosylation site is destroyed by substitution with any of the other 19 natural amino acids, but preferably a glutamine (Q), histidine (H), lysine (K) or arginine (R). Also preferably the mutation occurs at position C93, where the free cysteine is replaced by any of the other 19 natural amino acids, and preferably a serine (S). Also preferably the mutation occurs at position N101 or G102, where these 2 amino acids constitute an NG motif which can be prone to deamidation. Preferably, this potential NG motif can be destroyed by substitution of G102 with alanine (A) or proline (P). Also, trypophane (W) at position 67, which could be prone to oxidation, can be substituted with tyrosine (Y) or phenylalanine (F), and methionine (M) at positions 70 and 108 which could be prone to oxidation can be substituted with leucine (L), phenylalanine (F) or isoleucine (I).

sPD-1 or its fragments are used in the invention as X and Y.

By « sPD-1 fragment », it is meant a fragment of at least 140 contiguous amino acids, preferably at least 141, preferably at least 142, preferably at least 143 amino acids, and preferably of at most 145 amino acids, of sPD-1. Preferably, said fragment has a length of 143 amino acids. Preferably, the sPD-1 fragment is SEQ ID NO :3.

By « PD-1 ligand » or « ligand of PD-1 », it is meant PD-L1 and/or PD-L2.

### Identity

The percent amino acid sequence identity is defined as the percent of amino acid residues in a Compared Sequence that are identical to the Reference Sequence after aligning the sequences and introducing gaps if necessary, to achieve the maximum sequence identity. Alignment for purposes of determining percent amino acid sequence identity can be achieved in various ways known to a person of skill in the art, for instance using publicly available computer software such as BLAST (Altschul et al., J. Mol. Biol., 1990, 215, 403-). When using such software, the default parameters, e.g., for gap penalty and extension penalty, are preferably used. For amino acid sequences, the BLASTP program uses as default a word length (W) of 3 and an expectation (E) of 10. The percent identity is determined on at least 20 consecutive amino acid residues of the reference sequence.

### Linker

The term « linker » refers to any suitable peptide linker, such as a short peptide fragment. For example, a linker in accordance with the invention may comprise a short peptide, preferably from 1 to 30 amino acids, preferably from 2 to 25 amino acids. It typically comprises small amino acid residues or hydrophilic amino acid residues (e.g., glycine, serine, threonine, proline, aspartic acid, asparagine, etc.). One example of such a linker is Gly-Gly-Gly-Gly-Ser (G4S). Other examples may include permutations of these amino acids in the sequence, such as GGGSG, GGSGG, GSGGG or SGGGG. Further examples may include peptides containing amino acid residues other than G or S, such as GGTGS, GTSPGG or GNGGGS. One skilled in the art would appreciate that many commonly used peptide linkers may be used in embodiments of the invention. In accordance with some embodiments of the invention, the linkers may comprise repeat units to increase the linker length. For example, some linkers may comprise two G4S-repeated linkers, three G4S-repeated linkers, or four G4S-repeated linkers. Furthermore, some "repeat-like" linkers may comprise a mix of different peptide sequences - such as G4S - GGSGG - G4S - SGGGG.

Preferably, the linker is (GGGGS)ₚ, with p being an integer from 1 to 5, preferably from 2 to 4, preferably 3.

The sequences used in the present invention are detailed in the following table :

| **SE Q ID NO:** | **Definition** | **Sequence** |
|---|---|---|
| **1** | VL domain of anti-CD28 antibody clone 9.3 | DIELTQSPASLAVSLGQRATISCRASESVEYYVTSLM QWYQQKPGQPPKLLIFAASNVESGVPARFSGSGSGTN FSLNIHPVDEDDVAMYFCQQSRKVPYTFGGGTKLEIK |
| **2** | VH domain of anti-CD28 antibody clone 9.3 | EVKLQQSGPGLVTPSQSLSITCTVSGFSLSDYGVHWV RQSPGQGLEWLGVIWAGGGTNYNSALMSRKSISKDNS KSQVFLKMNSLQADDTAVYYCARDKGYSYYYSMDYWG QGTSVTVSS |
| **3** | Human wild-type soluble PD-1 protein fragment (i.e. X and/or Y) | LDSPDRPWNPPTFSPALLVVTEGDNATFTCSFSNTSE SFVLNWYRMSPSNQTDKLAAFPEDRSQPGQDCRFRVT QLPNGRDFHMSVVRARRNDSGTYLCGAISLAPKAQIK ESLRAELRVTERRAEVPTAHPSPSPRPAGQFQ |
| **4** | Mutated human soluble PD-1 protein fragment (i.e. with mutations G124S, K131Y and A132I in bold) (i.e. X and/or Y) | LDSPDRPWNPPTFSPALLVVTEGDNATFTCSFSNTSE SFVLNWYRMSPSNQTDKLAAFPEDRSQPGQDCRFRVT QLPNGRDFHMSVVRARRNDSGTYLCSAISLAPYIQIK ESLRAELRVTERRAEVPTAHPSPSPRPAGQFQ |
| **5** | Human light chain kappa constant domain (CL) | |
| **6** | Human IgG1 heavy chain constant domain (CH1-hinge-CH2-CH3) | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKRVEPKSCDKTHTCPP |
| | | CPAPEFEGGPSVFLFPPKPKDTLMISRTPEVTCVVVD VSHEDPEVKFNWYVDGVEVHNAKTKPREEQYNSTYR VVSVLTVLHQDWLNGKEYKCKVSNKALPASIEKTISK AKGQPREPQVYTLPPSREEMTKNQVSLTCLVKGFYPS DIAVEWESNGQPENNYKTTPPVLDSDGSFFLYSKLTV DKSRWQQGNVFSCSVMHEALHNHYTQKSLSLSP |
| **7** | VL-CL of a bispecific protein complex of the invention (i.e. A) | DIELTQSPASLAVSLGQRATISCRASESVEYYVTSLM QWYQQKPGQPPKLLIFAASNVESGVPARFSGSGSGTN FSLNIHPVDEDDVAMYFCQQSRKVPYTFGGGTKLEIK RTVAAPSVFIFPPSDEQLKSGTASVVCLLNNFYPREA KVQWKVDNALQSGNSQESVTEQDSKDSTYSLSSTLT LSKADYEKHKVYACEVTHQGLSSPVTKSFNRGEC |
| **8** | Anti-CD28 heavy chain fused by linker (underlined) to wild-type soluble PD-1 fragment of a bispecific protein complex of the invention (i.e. B-C-X) | EVKLQQSGPGLVTPSQSLSITCTVSGFSLSDYGVHWV RQSPGQGLEWLGVIWAGGGTNYNSALMSRKSISKDNS KSQVFLKMNSLQADDTAVYYCARDKGYSYYYSMDYWG QGTSVTVSSASTKGPSVFPLAPSSKSTSGGTAALGCL VKDYFPEPVTVSWNSGALTSGVHTFPAVLQSSGLYS LSSVVTVPSSSLGTQTYICNVNHKPSNTKVDKRVEPK SCDKTHTCPPCPAPEFEGGPSVFLFPPKPKDTLMISR TPEVTCVVVDVSHEDPEVKFNWYVDGVEVHNAKTKPR EEQYNSTYRVVSVLTVLHQDWLNGKEYKCKVSNKALP ASIEKTISKAKGQPREPQVYTLPPSREEMTKNQVSL TCLVKGFYPSDIAVEWESNGQPENNYKTTPPVLDSDG SFFLYSKLTVDKSRWQQGNVFSCSVMHEALHNHYTQK SLSLSPGGGGSGGGGSGGGGSLDSPDRPWNPPTFSPA LLVVTEGDNATFTCSFSNTSESFVLNWYRMSPSNQTD KLAAFPEDRSQPGQDCRFRVTQLPNGRDFHMSVVRA RRNDSGTYLCGAISLAPKAQIKESLRAELRVTERRAE VPTAHPSPSPRPAGQFQ |
| **9** | Anti-CD28 heavy chain fused by linker (underlined) to mutated soluble PD-1 fragment of a bispecific protein complex of the invention (i.e. B-C-X) | EVKLQQSGPGLVTPSQSLSITCTVSGFSLSDYGVHWV RQSPGQGLEWLGVIWAGGGTNYNSALMSRKSISKDNS KSQVFLKMNSLQADDTAVYYCARDKGYSYYYSMDYWG QGTSVTVSSASTKGPSVFPLAPSSKSTSGGTAALGCL VKDYFPEPVTVSWNSGALTSGVHTFPAVLQSSGLYS LSSVVTVPSSSLGTQTYICNVNHKPSNTKVDKRVEPK SCDKTHTCPPCPAPEFEGGPSVFLFPPKPKDTLMISR TPEVTCVVVDVSHEDPEVKFNWYVDGVEVHNAKTKPR EEQYNSTYRVVSVLTVLHQDWLNGKEYKCKVSNKALP ASIEKTISKAKGQPREPQVYTLPPSREEMTKNQVSL TCLVKGFYPSDIAVEWESNGQPENNYKTTPPVLDSDG SFFLYSKLTVDKSRWQQGNVFSCSVMHEALHNHYTQK SLSLSPGGGGSGGGGSGGGGSLDSPDRPWNPPTFSPA LLVVTEGDNATFTCSFSNTSESFVLNWYRMSPSNQTD KLAAFPEDRSQPGQDCRFRVTQLPNGRDFHMSVVRA |
| | | RRNDSGTYLCSAISLAPYIQIKESLRAELRVTERRAE VPTAHPSPSPRPAGOFO |
| 10 | Human wild-type soluble PD-1 protein | FLDSPDRPWNPPTFSPALLVVTEGDNATFTCSFSNTS ESFVLNWYRMSPSNQTDKLAAFPEDRSQPGQDCRFRV TQLPNGRDFHMSVVRARRNDSGTYLCGAISLAPKAQI KESLRAELRVTERRAEVPTAHPSPSPRPAGQFQTLV |
| 11 | H-CDR1 in Kabat of VH of clone 9.3 | DYGVH |
| 12 | H-CDR2 in Kabat of VH of clone 9.3 | VIWAGGGTNYNSALMS |
| 13 | H-CDR3 in Kabat of VH of clone 9.3 | DKGYSYYYSMDY |
| 14 | L-CDR1 in Kabat of VL of clone 9.3 | RASESVEYYVTSLMQ |
| 15 | L-CDR2 in Kabat of VL of clone 9.3 | AASNVES |
| 16 | L-CDR3 in Kabat (or IMGT) of VL of clone 9.3 | QQSRKVPYT |
| 17 | H-CDR1 in IMGT of VH of clone 9.3 | GFSLSDYG |
| 18 | H-CDR2 in IMGT of VH of clone 9.3 | IWAGGGT |
| 19 | H-CDR3 in IMGT of VH of clone 9.3 | ARDKGYSYYYSMDY |
| 20 | L-CDR1 in IMGT of VL of clone 9.3 | ESVEYYVTSL |
| 21 | LCDR2 in IMGT of VL of clone 9.3 | AA |
| 22 | H-CDR2 in Kabat of humanized VH of clone 9.3 | AIWAGGGTNYASSVMG |
| 23 | L-CDR1 in Kabat of humanized VL of clone 9.3 | RASESVEYYVTSLMA |
| 24 | H-CDR1 in IMGT of humanized VH of clone 9.3 | GFTFSDYG |
| 25 | VL domain of humanized anti-CD28 antibody clone 9.3 | DIVMTQSPDSLAVSLGERATINCRASESVEYYVTSLM AWYQQKPGQPPKLLIYAASNVESGVPDRFSGSGSGTN FSLTISSLQAEDVAVYYCQQSRKVPYTFGQGTKLEIK |
| 26 | VH domain of humanized anti-CD28 antibody clone 9.3 | EVQLVQSGGGLVQPGGSLRLSCAGSGFTFSDYGVHWV RQAPGKGLEWVSAIWAGGGTNYASSVMGRFTISRDNA KNSLYLQMNSLRAEDMAVYYCARDKGYSYYYSMDYWG QGTLVTVSS |
| 27 | C domain (hinge-CH2-CH3 domain) | EPKSCDKTHTCPPCPAPEFEGGPSVFLFPPKPKDTLM ISRTPEVTCVVVDVSHEDPEVKFNWYVDGVEVHNAKT KPREEQYNSTYRVVSVLTVLHQDWLNGKEYKCKVSNK ALPASIEKTISKAKGQPREPQVYTLPPSREEMTKNQV SLTCLVKGFYPSDIAVEWESNGQPENNYKTTPPVLD SDGSFFLYSKLTVDKSRWQQGNVFSCSVMHEALHNHY TQKSLSLSP |
| 28 | B domain | EVKLQQSGPGLVTPSQSLSITCTVSGFSLSDYGVHWV RQSPGQGLEWLGVIWAGGGTNYNSALMSRKSISKDNS KSQVFLKMNSLQADDTAVYYCARDKGYSYYYSMDYWG QGTSVTVSSASTKGPSVFPLAPSSKSTSGGTAALGCL VKDYFPEPVTVSWNSGALTSGVHTFPAVLQSSGLYS LSSVVTVPSSSLGTQTYICNVNHKPSNTKVDKRV |
| 29 | CH1 domain | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKRV |
| 30 | Fc fusion construct: A) 9.3 Light Chain Kappa 9.3 VL Human Kappa constant domain | DIELTQSPASLAVSLGQRATISCRASESVEYYVTSLM QWYQQKPGQPPKLLIFAASNVESGVPARFSGSGSGTN FSLNIHPVDEDDVAMYFCQQSRKVPYTFGGGTKLEIK RTVAAPSVFIFPPSDEQLKSGTASVVCLLNNFYPREA KVQWKVDNALQSGNSQESVTEQDSKDSTYSLSSTLT LSKADYEKHKVYACEVTHQGLSSPVTKSFNRGEC |
| 31 | Fc fusion construct with sPD-1 WT: B) 9.3 Heavy Chain IgG1-(GGGS)3 Linker-sPD1 WT 9.3 VH Human IgG1 = CH1-hinge-CH2-CH3 constant domains | EVKLQQSGPGLVTPSQSLSITCTVSGFSLSDYGVHWV RQSPGQGLEWLGVIWAGGGTNYNSALMSRKSISKDNS KSQVFLKMNSLQADDTAVYYCARDKGYSYYYSMDYWG QGTSVTVSSASTKGPSVFPLAPSSKSTSGGTAALGCL VKDYFPEPVTVSWNSGALTSGVHTFPAVLQSSGLYS LSSVVTVPSSSLGTQTYICNVNHKPSNTKVDKRVEPK SCDKTHTCPPCPAPEFEGGPSVFLFPPKPKDTLMISR TPEVTCVVVDVSHEDPEVKFNWYVDGVEVHNAKTKPR EEQYNSTYRVVSVLTVLHQDWLNGKEYKCKVSNKALP ASIEKTISKAKGQPREPQVYTLPPSREEMTKNQVSL TCLVKGFYPSDIAVEWESNGQPENNYKTTPPVLDSDG SFFLYSKLTVDKSRWQQGNVFSCSVMHEALHNHYTQK SLSLSPGGGGSGGGGSGGGGSLDSPDRPWNPPTFSPA LLVVTEGDNATFTCSFSNTSESFVLNWYRMSPSNQTD KLAAFPEDRSQPGQDCRFRVTQLPNGRDFHMSVVRA |
| | | RRNDSGTYLCGAISLAPKAQIKESLRAELRVTERRAE VPTAHPSPSPRPAGQFQ |
| 32 | Fc fusion construct with sPD-1 WT: C) Dummy Heavy Chain IgG1-(GGGS)3 Linker-sPD1 WT Human IgG1 = hinge-CH2-CH3 | DKTHTCPPCPAPEFEGGPSVFLFPPKPKDTLMISRTP EVTCVVVDVSHEDPEVKFNWYVDGVEVHNAKTKPREE QYNSTYRVVSVLTVLHQDWLNGKEYKCKVSNKALPAS IEKTISKAKGQPREPQVYTLPPSREEMTKNQVSLTCL VKGFYPSDIAVEWESNGQPENNYKTTPPVLDSDGSF FLYSKLTVDKSRWQQGNVFSCSVMHEALHNHYTQKSL SLSPGGGGSGGGGSGGGGSLDSPDRPWNPPTFSPALL VVTEGDNATFTCSFSNTSESFVLNWYRMSPSNQTDKL AAFPEDRSQPGQDCRFRVTQLPNGRDFHMSVVRARRN DSGTYLCGAISLAPKAQIKESLRAELRVTERRAEVP TAHPSPSPRPAGQFQ |
| 33 | Fc fusion construct with sPD-1 G124S/K131Y/A132I : B) 9.3 Heavy Chain IgG1-(GGGS)3 Linker-sPD1 (G124S/K131Y/A1321) 9.3 VH Human IgG1 = CH1-hinge-CH2-CH3 constant domains | EVKLQQSGPGLVTPSQSLSITCTVSGFSLSDYGVHWV RQSPGQGLEWLGVIWAGGGTNYNSALMSRKSISKDNS KSQVFLKMNSLQADDTAVYYCARDKGYSYYYSMDYWG QGTSVTVSSASTKGPSVFPLAPSSKSTSGGTAALGCL VKDYFPEPVTVSWNSGALTSGVHTFPAVLQSSGLYS LSSVVTVPSSSLGTQTYICNVNHKPSNTKVDKRVEPK SCDKTHTCPPCPAPEFEGGPSVFLFPPKPKDTLMISR TPEVTCVVVDVSHEDPEVKFNWYVDGVEVHNAKTKPR EEQYNSTYRVVSVLTVLHQDWLNGKEYKCKVSNKALP ASIEKTISKAKGQPREPQVYTLPPSREEMTKNQVSL TCLVKGFYPSDIAVEWESNGQPENNYKTTPPVLDSDG SFFLYSKLTVDKSRWQQGNVFSCSVMHEALHNHYTQK SLSLSPGGGGSGGGGSGGGGSLDSPDRPWNPPTFSPA LLVVTEGDNATFTCSFSNTSESFVLNWYRMSPSNQTD KLAAFPEDRSQPGQDCRFRVTQLPNGRDFHMSVVRA RRNDSGTYLCSAISLAPYIQIKESLRAELRVTERRAE VPTAHPSPSPRPAGQFQ |
| 34 | Fc fusion construct with sPD-1 | DKTHTCPPCPAPEFEGGPSVFLFPPKPKDTLMISRTP EVTCVVVDVSHEDPEVKFNWYVDGVEVHNAKTKPREE QYNSTYRVVSVLTVLHQDWLNGKEYKCKVSNKALPAS IEKTISKAKGQPREPQVYTLPPSREEMTKNQVSLTCL VKGFYPSDIAVEWESNGQPENNYKTTPPVLDSDGSF FLYSKLTVDKSRWQQGNVFSCSVMHEALHNHYTQKSL SLSPGGGGSGGGGSGGGGSLDSPDRPWNPPTFSPALL VVTEGDNATFTCSFSNTSESFVLNWYRMSPSNQTDKL AAFPEDRSQPGQDCRFRVTQLPNGRDFHMSVVRARRN DSGTYLCSAISLAPYIQIKESLRAELRVTERRAEVP TAHPSPSPRPAGQFQ |
| | G124S/K131Y/A132I : | |
| | C) Dummy Heavy Chain IgG1-(GGGS)3 Linker-sPD1(G124S/K131Y/A13 2I) | |
| | Human IgG1 = hinge-CH2-CH3 | |
| 35 | H-CDR1 in Kabat of VH of an anti-CD28 antibody | GGSISSYY |
| 36 | H-CDR2 in Kabat of VH of an anti-CD28 antibody | IYYSGIT |
| 37 | H-CDR3 in Kabat of VH of an anti-CD28 antibody | ARWGVRRDYYYYGMDV |
| 38 | L-CDR1 in Kabat of VL of an anti-CD28 antibody | QSVSSSY |
| 39 | L-CDR2 in Kabat of VL of an anti-CD28 antibody | GAS |
| 40 | L-CDR3 in Kabat of VL of an anti-CD28 antibody | QQYGSSPWT |
| 41 | VL of an anti-CD28 antibody | |
| 42 | VH of an anti-CD28 antibody | EIVLTQSPGTLSLSPGERATLSCRASQSVSSSYLAWY QQKPGQAPRLLIYGASSRATGIPDRFSGSGSGTDFTL TISRLEPEDFAVYYCQQYGSSPWTFGQGTKVEIK QVQLQESGPGLVKPSETLSLTCTVSGGSISSYYWSWI RQPPGKGLEWIGYIYYSGITHYNPSLKSRVTISVDTSKI QFSLKLSSVTAADTAVYYCARWGVRRDYYYYGMDV WGQGTTVTVSS |
| 43 | H-CDR1 in Kabat of VH of an anti-CD28 antibody | GFTFSSYG |
| 44 | H-CDR1 in Kabat of VH of an anti-CD28 antibody | GFTFSRNN |
| 45 | H-CDR2 in Kabat of VH of an anti-CD28 antibody | ISYAGNNK |
| 46 | H-CDR2 in Kabat of VH of an anti-CD28 antibody | ISSNGGRT |
| 47 | H-CDR3 in Kabat of VH of an anti-CD28 antibody | AKDSYYDFLTDPDVLDI |
| 48 | H-CDR3 in Kabat of VH of an anti-CD28 antibody | TRDDELLSFDY |
| 49 | L-CDR1 in Kabat of VL of an anti-CD28 antibody | QSISSY |
| 50 | L-CDR2 in Kabat of VL of an anti-CD28 antibody | AAS |
| 51 | L-CDR3 in Kabat of VL of an anti-CD28 antibody | QQSYSTPPIT |
| 52 | VH of an anti-CD28 antibody | QVQLQESGPGLVKPSETLSLTCTVSGGSISSYYWSWI RQPPGKGLEWIGYIYYSGITHYNPSLKSRVTISVDTSKI QFSLKLSSVTAADTAVYYCARWGVRRDYYYYGMDV WGQGTTVTVSS |
| 53 | VL of an anti-CD28 antibody | EIVLTQSPGTLSLSPGERATLSCRASQSVSSSYLAWY QQKPGQAPRLLIYGASSRATGIPDRFSGSGSGTDFTL TISRLEPEDFAVYYCQQYGSSPWTFGQGTKVEIK |
| 54 | VH of an anti-CD28 antibody | QVQLVESGGGVVQPGRSLRLSCAASGFTFSSYGMH WVRQAPGKGLEWVAVISYAGNNKYYADSVKGRFTVS RDNSKKTLYLQMNSLRSEDTAVYYCAKDSYYDFLTD PDVLDIWGQGTMVTVSS |
| 55 | VL of an anti-CD28 antibody | DIQMTQSPSSLSASVGDRVTITCRASQSISSYLNWYQ QKPGKAPKLLIYAASSLQSGVPSRFSGSGSGTDFTLTI SSLQPEDFATYYCQQSYSTPPITFGQGTRLEIK EVQLVESGGGLVQPGGSLRLSCAASGFTFSRNNMH |
| 56 | VH of an anti-CD28 antibody | EVQLVESGGGLVQPGGSLRLSCAASGFTFSRNNMH WVRQAPGKGLEYVSGISSNGGRTYYADSVKGRFTIS RDNSKNTLYLQMGGLRAADMAVYFCTRDDELLSFDY WGQGTLVTVSS |
| 57 | H-CDR1 in Kabat of VH of an anti-CD28 antibody | GYTFTSYY |
| 58 | H-CDR2 in Kabat of VH of an anti-CD28 antibody | IYPGNVNT |
| 59 | H-CDR3 in Kabat of VH of an anti-CD28 antibody | TRSHYGLDWNFDV |
| 60 | L-CDR1 in Kabat of VL of an anti-CD28 antibody | QNIYVW |
| 61 | L-CDR2 in Kabat of VL of an anti-CD28 antibody | KAS |
| 62 | L-CDR3 in Kabat of VL of an anti-CD28 antibody | QQGQTYPY |
| 63 | VL of an anti-CD28 antibody | DIQMTQSPSSLSASVGDRVTITCQASQNIYVWLNWYQQ KPGKAPKLLIYKASNLHTGVPSRFSGSGSGTDFTLTISSL QPEDIATYYCQQGQTYPYTFGQGTKLEIK QVQLVQSGAEVVKPGASVKVSCKASGYTFTSYYIHWVR QAPGQGLEWIGSIYPGNVNTNYAQKFQGRATLTVDTSIS TAYMELSRLRSDDTAVYYCTRSHYGLDWNFDVWGKGT TVTVSS |
| 64 | VH of an anti-CD28 antibody | QVQLVQSGAEVVKPGASVKVSCKASGYTFTSYYIHWVR QAPGQGLEWIGSIYPGNVNTNYAQKFQGRATLTVDTSIS TAYMELSRLRSDDTAVYYCTRSHYGLDWNFDVWGKGT TVTVSS |
| 65 | H-CDR1 in Kabat of VH of clone 1A7[CD28] H1L1 | SYAMS |
| 66 | H-CDR2 in Kabat of VH of clone 1A7[CD28] H1L1 | TISGSGDSTYYADSVKG |
| 67 | H-CDR3 in Kabat of VH of clone 1A7[CD28] H1L1 | SGPGLRQVGFDY |
| 68 | L-CDR1 in Kabat of VL of clone 1A7[CD28] H1L1 | RASQSISSYLN |
| 69 | L-CDR2 in Kabat of VL of clone 1A7[CD28] H1L1 | AASSLQS |
| 70 | L-CDR3 in Kabat of VL of clone 1A7[CD28] H1L1 | QQSYSTPFT |
| 71 | L-CDR3 in Kabat of VL of clone 1A7[CD28] H1 L1.71 | QQVYSTPFT |
| 72 | H-CDR1 in Kabat of VH of clone 1A7[CD28] H1.1 L1.71 | SYYMS |
| 73 | H-CDR2 in Kabat of VH of clone 1A7[CD28] H1.14 L1 | TISESGDSTYYADSVKG |

### Bispecific protein complex of the invention

The bispecific protein complex according to the invention has the following formula :
A:B-C-X:(D)ₘ-C'-Y wherein:
A is chosen from VL-(CL)ₙ, VL-(CH1)ₙ and VH, wherein
   VL is a light chain variable region of an antibody directed against CD28,
   CL is a light chain constant region of an antibody,
   CH1 is the first domain of a heavy chain constant region of an antibody, and VH is the heavy chain variable region of an antibody directed against CD28 which
   forms with VL a binding site to CD28, and
n is an integer which is 0 or 1,
B-C-X is a first fusion protein, wherein :
   B is chosen from VH-(CH1)n, VH-(CL)n and VL, wherein VH, CH1, CL, VL and n are as defined for A,
   C is the hinge-CH2-CH3 domain of an antibody,
   X is a soluble PD-1 protein or one of its fragments, and
   the C-terminal end of the CH3 domain of C is linked to the N-terminal end of X ; with the proviso than when A is VH, then B is VL, and with the proviso than when n is 1, then A is VL-CL and B is VH-CH1, or A is VL-CH1 and B is VH-CL,
(D)ₘ-C'-Y is a second fusion protein, wherein :
   D is a protein or a protein dimer,
   m is an integer which is 0 or 1,
   C' is a sequence which shows at least 80% identity, preferably at least 85% identity, preferably at least 90% identity, preferably at least 95% identity with C ;
   Y is a soluble PD-1 protein or one of its fragments, and Y is identical to or different from X, and
   the C-terminal end of the CH3 domain of C' is linked to the N-terminal end of Y; and
the symbol ":" of the formula is a binding interaction between A and B, or between C of the first fusion protein and C' of the second fusion protein.

It has to be noted that each symbol "-" in the above formula is a covalent bond or a linker. Preferably, it is a covalent bond.

Preferably, the binding interaction ":" is chosen from a disulfide bond and a linker.

Preferably, the binding interactions ":" respectively between A and B, and between C of the first fusion protein and C' of the second fusion protein, are disulfide bonds. In this case, the bispecific protein complex of the invention is a trimer, i.e. comprises proteins A, B-C-X and (D)ₘ-C'-Y.

Alternatively, the binding interactions ":" respectively between A and B, and between C of the first fusion protein and C' of the second fusion protein, are linkers. Especially, preferably, the binding interaction ":" between A and B is a linker. In this case, the bispecific protein complex of the invention comprises a scFv as A:B.

The invention also relates to a bispecific protein complex having the formula B-C-X:(D)ₘ-C'-Y wherein:
B-C-X is a first fusion protein wherein:
B is chosen from VHH-(CH1)ₙ, VHH-(CL)ₙ and VHH, wherein CL is a light chain constant region of an antibody,
CH1 is the first domain of a heavy chain constant region of an antibody, and
VHH is a single heavy chain variable region of a Camelid antibody (preferably a llama VHH) directed against CD28 and which binds CD28, and
n is an integer which is 0 or 1,
C is the hinge-CH2-CH3 domain of an antibody,
X is a soluble PD-1 protein or one of its fragments, and
the C-terminal end of the CH3 domain of C is linked to the N-terminal end of X ; and (D)ₘ-C'-Y is a second fusion protein, wherein :
   D is a protein or a protein dimer,
   m is an integer which is 0 or 1,
   C' is a sequence which shows at least 80% identity, preferably at least 85% identity, preferably at least 90% identity, preferably at least 95% identity with C ;
   Y is a soluble PD-1 protein or one of its fragments, and Y is identical to or different from X, and
   the C-terminal end of the CH3 domain of C' is linked to the N-terminal end of Y ;
: is a binding interaction respectively between C of the first fusion protein and C' of the second fusion protein.

Preferably, the anti-CD28 antibody light and heavy chains comprise the following first combination of 6 CDRs, as in Kabat:
H-CDR1: DYGVH (SEQ ID NO: 11)
H-CDR2: VIWAGGGTNYNSALMS (SEQ ID NO: 12)
H-CDR3: DKGYSYYYSMDY (SEQ ID NO: 13),
and
L-CDR1: RASESVEYYVTSLMQ (SEQ ID NO: 14)
L-CDR2: AASNVES (SEQ ID NO: 15)
L-CDR3: QQSRKVPYT (SEQ ID NO: 16), or
the anti-CD28 antibody light and heavy chains comprise the following second combination of 6 CDRs, as in Kabat:
   H-CDR1: DYGVH (SEQ ID NO: 11)
   H-CDR2: AIWAGGGTNYASSVMG (SEQ ID NO: 22)
   H-CDR3: DKGYSYYYSMDY (SEQ ID NO: 13),
   and
   L-CDR1: RASESVEYYVTSLMA (SEQ ID NO: 23)
   L-CDR2: AASNVES (SEQ ID NO: 15)
   L-CDR3: QQSRKVPYT (SEQ ID NO: 16), or
the anti-CD28 antibody light and heavy chains comprise the following third combinations of 6 CDRs, as in Kabat:
   H-CDR1: GGSISSYY (SEQ ID NO:35)
   H-CDR2: IYYSGIT (SEQ ID NO:36)
   H-CDR3: ARWGVRRDYYYYGMDV (SEQ ID NO:37),
   and
   L-CDR1: QSVSSSY (SEQ ID NO:38)
   L-CDR2: GAS (SEQ ID NO:39)
   L-CDR3: QQYGSSPWT (SEQ ID NO:40), or
the anti-CD28 antibody light and heavy chains comprise the following fourth combinations of 6 CDRs, as in Kabat:
   H-CDR1 chosen from GFTFSSYG (SEQ ID NO:43) and GFTFSRNN (SEQ ID NO:44)
   H-CDR2 chosen from ISYAGNNK (SEQ ID NO:45) and ISSNGGRT (SEQ ID NO:46)
   H-CDR3 chosen from AKDSYYDFLTDPDVLDI (SEQ ID NO:47) and TRDDELLSFDY (SEQ ID NO:48),
   and
   L-CDR1: QSISSY (SEQ ID NO:49)
   L-CDR2: AAS (SEQ ID NO:50)
   L-CDR3: QQSYSTPPIT (SEQ ID NO:51), or
the anti-CD28 antibody light and heavy chains comprise the following fifth combinations of 6 CDRs, as in Kabat:
   H-CDR1: GFTFSRNN (SEQ ID NO:44)
   H-CDR2: ISSNGGRT (SEQ ID NO:46)
   H-CDR3: TRDDELLSFDY (SEQ ID NO:48),
   and
   L-CDR1: QSISSY (SEQ ID NO:49)
   L-CDR2: AAS (SEQ ID NO:50)
   L-CDR3: QQSYSTPPIT (SEQ ID NO:51), or
the anti-CD28 antibody light and heavy chains comprise the following sixth combinations of 6 CDRs, as in Kabat:
   H-CDR1: GYTFTSYY (SEQ ID NO:57)
   H-CDR2: IYPGNVNT (SEQ ID NO:58)
   H-CDR3: TRSHYGLDWNFDV (SEQ ID NO:59),
   and
   L-CDR1: QNIYVW (SEQ ID NO:60)
   L-CDR2: KAS (SEQ ID NO:61)
   L-CDR3: QQGQTYPY (SEQ ID NO:62).

Alternatively, preferably, the anti-CD28 antibody light and heavy chains comprise one of the following combinations of 6 CDRs, as in Kabat:
H-CDR1: SYAMS (SEQ ID NO:65)
H-CDR2: TISGSGDSTYYADSVKG (SEQ ID NO:66)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQSYSTPFT (SEQ ID NO:70),
or
H-CDR1: SYAMS (SEQ ID NO:65)
H-CDR2: TISGSGDSTYYADSVKG (SEQ ID NO:66)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQVYSTPFT (SEQ ID NO:71),
or
H-CDR1: SYYMS (SEQ ID NO:72)
H-CDR2: TISGSGDSTYYADSVKG (SEQ ID NO:66)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQVYSTPFT (SEQ ID NO:71),
or
H-CDR1: SYYMS (SEQ ID NO:72)
H-CDR2: TISESGDSTYYADSVKG (SEQ ID NO:73)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQSYSTPFT (SEQ ID NO:70),
or
H-CDR1: SYYMS (SEQ ID NO:72)
H-CDR2: TISESGDSTYYADSVKG (SEQ ID NO:73)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQVYSTPFT (SEQ ID NO:71).

Preferably, VL comprises, preferably consists in, the VL domain of anti-CD28 antibody clone 9.3. Preferably, VL comprises, preferably consists in, the sequence SEQ ID NO :1.

Preferably, VH comprises, preferably consists in, the VH domain of anti-CD28 antibody clone 9.3. Preferably, VH comprises, preferably consists in, the sequence SEQ ID NO :2.

Accorfding to another embodiment, preferably, VL comprises, preferably consists in, the VL domain of humanized anti-CD28 antibody clone 9.3. Preferably, VL comprises, preferably consists in, the sequence SEQ ID NO :25. Preferably, VH comprises, preferably consists in, the VH domain of humanized anti-CD28 antibody clone 9.3. Preferably, VH comprises, preferably consists in, the sequence SEQ ID NO:26.

Preferably, the VH and the VL bind CD28 and comprise the first following combination of 6 CDRs :
as in Kabat:
H-CDR1: DYGVH (SEQ ID NO: 11)
H-CDR2: VIWAGGGTNYNSALMS (SEQ ID NO: 12)
H-CDR3: DKGYSYYYSMDY (SEQ ID NO: 13),
and
L-CDR1: RASESVEYYVTSLMQ (SEQ ID NO: 14)
L-CDR2: AASNVES (SEQ ID NO: 15)
L-CDR3: QQSRKVPYT (SEQ ID NO: 16).

Said first combination of CDRs is as follows in IMGT :
H-CDR1: GFSLSDYG (SEQ ID NO: 17)
H-CDR2: IWAGGGT (SEQ ID NO: 18)
H-CDR3: ARDKGYSYYYSMDY (SEQ ID NO: 19),
and
L-CDR1: ESVEYYVTSL (SEQ ID NO: 20)
L-CDR2: AA (SEQ ID NO: 21)
L-CDR3: QQSRKVPYT (SEQ ID NO: 16).

Alternatively, more preferably, the VH and the VL bind CD28 and comprise the second following combination of 6 CDRs :
as in Kabat:
H-CDR1: DYGVH (SEQ ID NO: 11)
H-CDR2: AIWAGGGTNYASSVMG (SEQ ID NO: 22)
H-CDR3: DKGYSYYYSMDY (SEQ ID NO: 13),
and
L-CDR1: RASESVEYYVTSLMA (SEQ ID NO: 23)
L-CDR2: AASNVES (SEQ ID NO: 15)
L-CDR3: QQSRKVPYT (SEQ ID NO: 16).

Said second combination of CDRs is as follows in IMGT :
H-CDR1: GFTFSDYG (SEQ ID NO: 24)
H-CDR2: IWAGGGT (SEQ ID NO: 18)
H-CDR3: ARDKGYSYYYSMDY (SEQ ID NO: 19),
and
L-CDR1: ESVEYYVTSL (SEQ ID NO: 20)
L-CDR2: AA (SEQ ID NO: 21)
L-CDR3: QQSRKVPYT (SEQ ID NO: 16).

Preferably, CL comprises, preferably consists in, the CL domain of anti-CD28 antibody clone 9.3. Preferably, CL comprises, preferably consists in, the sequence SEQ ID NO :5.

According to an embodiment, A is VH and then B is VL. In this embodiment, the binding interaction ":" between A and B is preferably a linker. In this case, A:B is a scFv.

According to an other embodiment, n is 0, and A is VL. In this case, B is VH. In this other embodiment, the binding interaction ":" between A and B is also preferably a linker. In this case too, A:B is a scFv. Preferable said scFv comprises the sequence SEQ ID NO:1 fused to the sequence SEQ ID NO:2 with a linker.

According to a last embodiment, n is 1. Then, the proviso states that either (i) A is VL-CL and then B is VH-CH1, or (ii) A is VL-CH1 and then B is VH-CL. In this embodiment, the binding interaction ":" between A and B is preferably a disulfide bond. According to (i), A:B is a Fab fragment. According to (ii), CL and CH1 are permuted among the Fab fragment. Preferably, A is VL-CL of the sequence SEQ ID NO:7.

Preferably:
- n is 1;
- A is VL-CL of the sequence SEQ ID NO:7;
- B is VH-CH1;
- A:B is a Fab fragment.

Preferably, the second fusion protein (D)ₘ-C'-Y is such that m is 0. In this case, said second fusion protein is C'-Y. The formula is then A:B-C-X:C'-Y. This corresponds to the monovalent format of the bispecific protein complex of the invention. Indeed, the bispecific protein complex comprises only one binding site for CD28, which is A:B.

Alternatively, preferably, the second fusion protein (D)ₘ-C'-Y is such that m is 1. In this case, said second fusion protein is D-C'-Y. This corresponds to the bivalent format of the bispecific protein complex of the invention. Indeed, the bispecific protein complex comprises two binding sites for CD28, which are A:B and D.

C' is a sequence which shows at least 80% identity, preferably at least 85% identity, preferably at least 90% identity, preferably at least 95% identity with C. Preferably, C' shows at least 96%, preferably at least 97%, preferably at least 98%, preferably at least 99% identity with C. Preferably C' is identical to C. Preferably the binding interaction between C of the first fusion protein and C' of the second fusion protein, is a disulfide bond.

Preferably, C (hinge-CH2-CH3 domain of an antibody) is the sequence SEQ ID NO:27.

Each of X and Y is a soluble PD-1 protein or one of its fragments. Preferably, each of X and Y is chosen from wild-type soluble PD-1 proteins and their fragments, and soluble PD-1 proteins comprising at least one mutation and their fragments. Preferably, each of X and Y is chosen from the human wild-type soluble PD-1 protein and its fragments of 140 to 145 amino acids, and human soluble PD-1 proteins comprising at least one mutation as compared to the wild-type and their fragments of 140 to 145 amino acids.

Said mutation preferably increases the affinity of PD-1 to PD-L1 and/or PD-L2 as compared to the wild-type protein. Preferably, said mutation is a substitution, which is preferably selected from G124S, K131Y, A132I, A132V, A132L, V64H, N66I, N66V, Y68H, M70E, M70I, N74G, T76P, K78T, S87G, S87W, C93A, N116S, L122V, A125V, S127V, S127A, K135M, A140V and their combinations, wherein the amino acid numbering is the one of human wild-type full-length PD-1 protein, and provided that when at least two mutations are present, they do not occur on the same position.

Preferably, each of X and Y comprises the combinations of mutations G124S/K131Y/A132I, wherein the amino acid numbering is the one of human wild-type full-length PD-1 protein.

According to an embodiment, X and Y are different. For example, X is a wild-type sPD-1 or one of its fragments, and Y is a mutated sPD-1 or one of its fragments.

Preferably, X and Y are identical.

Preferably, X and Y are chosen from sequences SEQ ID NO:3, SEQ ID NO:4 and SEQ ID NO:10.

Preferably VL, VH, CH1 and hinge-CH2-CH3 domain belong to one single antibody directed against CD28. Preferably the CH1 domain is the sequence SEQ ID NO:29.

Preferably, the bispecific protein complex of the invention is such that m is 0. Thus preferably, the bispecific protein complex of the invention has the formula A:B-C-X:C'-Y wherein:
- A is VL-CL, wherein VL is a light chain variable region of an antibody directed against CD28,
- B is VH-CH1, wherein VH is the heavy chain variable region of an antibody directed against CD28 which forms with VL a binding site to CD28, preferably B is the sequence SEQ ID NO:28,
- C is the hinge-CH2-CH3 domain of an antibody,
- the binding interactions ":" are disulfide bonds,
- C' is identical to C, and
- X and Y are identical, and preferably chosen from sequences SEQ ID NO:3 and SEQ ID NO:4.

Preferably, the bispecific protein complex of the invention has the formula A:B-C-X:C'-Y (i.e. m is 0) wherein:
- A is of SEQ ID NO:7,
- the first fusion protein B-C-X is of SEQ ID NO:8 or SEQ ID NO:9,
- the binding interactions ":" are disulfide bonds,
- C' is a sequence which shows at least 95% identity with C, preferably C' is identical to C, and
- Y is a soluble PD-1 protein or one of its fragments, and Y is identical to or different from X, preferably Y is identical to X.

Preferably, C is the sequence SEQ ID NO:27.

Preferably, the bispecific protein complex of the invention has the formula A:B-C-X:C'-Y wherein:
- A is VL-CL, wherein VL is a light chain variable region of an antibody directed against CD28,
- B is VH-CH1, wherein VH is the heavy chain variable region of an antibody directed against CD28 which forms with VL a binding site to CD28,
- C is the hinge-CH2-CH3 domain of an antibody,
- the binding interactions ":" are disulfide bonds,
- C' is identical to C, and
- X and Y are identical, and preferably chosen from sequences SEQ ID NO:3 and SEQ ID NO:4, and
   said VL and VH comprise the following first combination of 6 CDRs as in Kabat:
   H-CDR1: DYGVH (SEQ ID NO: 11)
   H-CDR2: VIWAGGGTNYNSALMS (SEQ ID NO: 12)
   H-CDR3: DKGYSYYYSMDY (SEQ ID NO: 13),
   and
   L-CDR1: RASESVEYYVTSLMQ (SEQ ID NO: 14)
   L-CDR2: AASNVES (SEQ ID NO: 15)
   L-CDR3: QQSRKVPYT (SEQ ID NO: 16).
Said first combination of CDRs is as follows in IMGT :
H-CDR1: GFSLSDYG (SEQ ID NO: 17)
H-CDR2: IWAGGGT (SEQ ID NO: 18)
H-CDR3: ARDKGYSYYYSMDY (SEQ ID NO: 19),
and
L-CDR1: ESVEYYVTSL (SEQ ID NO: 20)
L-CDR2: AA (SEQ ID NO: 21)
L-CDR3: QQSRKVPYT (SEQ ID NO: 16).

Preferably, the bispecific protein complex of the invention has the formula A:B-C-X:C'-Y wherein:
- A is VL-CL, wherein VL is a light chain variable region of an antibody directed against CD28,
- B is VH-CH1, wherein VH is the heavy chain variable region of an antibody directed against CD28 which forms with VL a binding site to CD28,
- C is the hinge-CH2-CH3 domain of an antibody,
- the binding interactions ":" are disulfide bonds,
- C' is identical to C, and
- X and Y are identical, and preferably chosen from sequences SEQ ID NO:3 and SEQ ID NO:4, and
   said VL and VH comprise the following second combination of 6 CDRs as in Kabat:
   H-CDR1: DYGVH (SEQ ID NO: 11)
   H-CDR2: AIWAGGGTNYASSVMG (SEQ ID NO: 22)
   H-CDR3: DKGYSYYYSMDY (SEQ ID NO: 13),
   and
   L-CDR1: RASESVEYYVTSLMA (SEQ ID NO: 23)
   L-CDR2: AASNVES (SEQ ID NO: 15)
   L-CDR3: QQSRKVPYT (SEQ ID NO: 16).
Said second combination of CDRs is as follows in IMGT :
H-CDR1: GFTFSDYG (SEQ ID NO: 24)
H-CDR2: IWAGGGT (SEQ ID NO: 18)
H-CDR3: ARDKGYSYYYSMDY (SEQ ID NO: 19),
and
L-CDR1: ESVEYYVTSL (SEQ ID NO: 20)
L-CDR2: AA (SEQ ID NO: 21)
L-CDR3: QQSRKVPYT (SEQ ID NO: 16).

Preferably, the bispecific protein complex of the invention has the formula A:B-C-X:C'-Y wherein:
- A is VL-CL, wherein VL is a light chain variable region of an antibody directed against CD28,
- B is VH-CH1, wherein VH is the heavy chain variable region of an antibody directed against CD28 which forms with VL a binding site to CD28,
- C is the hinge-CH2-CH3 domain of an antibody,
- the binding interactions ":" are disulfide bonds,
- C' is identical to C, and
- X and Y are identical, and preferably chosen from sequences SEQ ID NO:3 and SEQ ID NO:4, and
   said VL and VH are respectively of sequences SEQ ID NO:25 and SEQ ID NO:26.

As shown in the examples, the monovalent construct of the invention (thus comprising only one Fab) retains ability to bind CD28 and subsequently to induce CD28 costimulatory pathway, resulting in T cell activation and proliferation. This is surprising, because prior publications showed that only Fab formats were able to produce this effect.

When m is 1, D is present in the second fusion protein. In this case, the formula of the bispecific protein complex is A:B-C-X:D-C'-Y.

D is a protein or a protein dimer. By "protein", it is meant an amino acid sequence of at least 50 amino acids, preferably at least 60 amino acids. Preferably, the protein comprises less than 300 amino acids, preferably less than 270 amino acids. By "protein dimer", it is meant a set of two proteins, which are linked together by a disulfide bond. Preferably the protein dimer comprises from 200 to 700 amino acids, preferably from 300 to 600 amino acids.The two proteins of the protein dimer may be identical (protein homodimer) or different (protein heterodimer).

As the protein complex of the invention is bispecific, D is preferably a sequence able to bind CD28.

Preferably, D is a protein, preferably a scFv that binds CD28. Preferably, according to an other embodiment, D is a protein dimer, preferably a protein heterodimer that binds CD28, more preferably a Fab fragment that binds CD28. Preferably, the VL-CL and VH-CH1 chains of said Fab fragment that binds CD28 are identical to A:B. In other words, preferably, D is a protein dimer D1:D2, wherein D1 and D2 are linked by a disulfide bond and form together a binding site to CD28. Preferably, D1 is VL-CL and D2 is VH-CH1, and the C-terminal end of D1 or D2 is fused to the N-teminal end of C'-Y.

Preferably, the bispecific protein complex of the invention is such that m is 1. Preferably, the bispecific protein complex of the invention has the formula A:B-C-X:D-C'-Y wherein:
- A is VL-CL,
- B is VH-CH1,
- the binding interactions ":" are disulfide bonds,
- C' is identical to C,
- D is a Fab fragment that binds CD28, and
- X and Y are identical, and preferably chosen from sequences SEQ ID NO:3 and SEQ ID NO:4.

Preferably, the bispecific protein complex of the invention has the formula A:B-C-X:D-C'-Y wherein:
- A is VL-CL,
- B is VH-CH1,
- the binding interactions ":" are disulfide bonds,
- C' is identical to C,
- D is a Fab fragment that binds CD28, wherein the VL-CL and VH-CH1 chains of said Fab fragment are identical to A:B, and
- X and Y are identical, and preferably chosen from sequences SEQ ID NO:3 and SEQ ID NO:4.

Preferably, the bispecific protein complex of the invention is such that:
- A is of SEQ ID NO:7,
- the first fusion protein B-C-X is of SEQ ID NO:8 or SEQ ID NO:9,
- the binding interactions ":" are disulfide bonds,
- the second fusion protein D-C'-Y is of sequence SEQ ID NO:8 or SEQ ID NO:9, wherein said sequence is linked in its N-terminal region by a disulfide bond to a second sequence SEQ ID NO:7, and
- X and Y are identical, and preferably chosen from sequences SEQ ID NO:3 and SEQ ID NO:4.

Preferably, the bispecific protein complex of the invention has the formula A:B-C-X:D-C'-Y wherein:
- A is VL-CL,
- B is VH-CH1,
- the binding interactions ":" are disulfide bonds,
- C' is identical to C,
- D is a Fab fragment that binds CD28, wherein the VL-CL and VH-CH1 chains of said Fab fragment are identical to A:B, and
- X and Y are identical, and preferably chosen from sequences SEQ ID NO:3 and SEQ ID NO:4, and
   said VL and VH comprise the following first combination of 6 CDRs as in Kabat:
   H-CDR1: DYGVH (SEQ ID NO: 11)
   H-CDR2: VIWAGGGTNYNSALMS (SEQ ID NO: 12)
   H-CDR3: DKGYSYYYSMDY (SEQ ID NO: 13),
   and
   L-CDR1: RASESVEYYVTSLMQ (SEQ ID NO: 14)
   L-CDR2: AASNVES (SEQ ID NO: 15)
   L-CDR3: QQSRKVPYT (SEQ ID NO: 16).
Said first combination of CDRs is as follows in IMGT :
H-CDR1: GFSLSDYG (SEQ ID NO: 17)
H-CDR2: IWAGGGT (SEQ ID NO: 18)
H-CDR3: ARDKGYSYYYSMDY (SEQ ID NO: 19),
and
L-CDR1: ESVEYYVTSL (SEQ ID NO: 20)
L-CDR2: AA (SEQ ID NO: 21)
L-CDR3: QQSRKVPYT (SEQ ID NO: 16).

Preferably, the bispecific protein complex of the invention has the formula A:B-C-X:D-C'-Y wherein:
- A is VL-CL,
- B is VH-CH1,
- the binding interactions ":" are disulfide bonds,
- C' is identical to C,
- D is a Fab fragment that binds CD28, wherein the VL-CL and VH-CH1 chains of said Fab fragment are identical to A:B, and
- X and Y are identical, and preferably chosen from sequences SEQ ID NO:3 and SEQ ID NO:4, and
   said VL and VH comprise the following second combination of 6 CDRs as in Kabat:
   H-CDR1: DYGVH (SEQ ID NO: 11)
   H-CDR2: AIWAGGGTNYASSVMG (SEQ ID NO: 22)
   H-CDR3: DKGYSYYYSMDY (SEQ ID NO: 13),
   and
   L-CDR1: RASESVEYYVTSLMA (SEQ ID NO: 23)
   L-CDR2: AASNVES (SEQ ID NO: 15)
   L-CDR3: QQSRKVPYT (SEQ ID NO: 16).
Said second combination of CDRs is as follows in IMGT :
H-CDR1: GFTFSDYG (SEQ ID NO: 24)
H-CDR2: IWAGGGT (SEQ ID NO: 18)
H-CDR3: ARDKGYSYYYSMDY (SEQ ID NO: 19),
and
L-CDR1: ESVEYYVTSL (SEQ ID NO: 20)
L-CDR2: AA (SEQ ID NO: 21)
L-CDR3: QQSRKVPYT (SEQ ID NO: 16).

Preferably, the bispecific protein complex of the invention has the formula A:B-C-X:D-C'-Y wherein:
- A is VL-CL,
- B is VH-CH1,
- the binding interactions ":" are disulfide bonds,
- C' is identical to C,
- D is a Fab fragment that binds CD28, wherein the VL-CL and VH-CH1 chains of said Fab fragment are identical to A:B, and
- X and Y are identical, and preferably chosen from sequences SEQ ID NO:3 and SEQ ID NO:4, and
   said VL and VH are respectively of sequences SEQ ID NO:25 and SEQ ID NO:26.

This format corresponds to a bivalent construct.

Said differently, said format is a bispecific protein complex comprising an anti-CD28 antibody, wherein each C-terminal end of the CH3 domain of the heavy chain of the antibody is linked to the N-terminal end of a soluble PD-1 protein or one of its fragments. Preferably, each soluble PD-1 protein or one of its fragments is chosen from sequences SEQ ID NO:3 and SEQ ID NO:4. Preferably, said bispecific protein complex comprises an anti-CD28 antibody, wherein each C-terminal end of the CH3 domain of the heavy chain of the antibody is linked to the N-terminal end of a sequence SEQ ID NO:3. Preferably, said bispecific protein complex comprises an anti-CD28 antibody, wherein each C-terminal end of the CH3 domain of the heavy chain of the antibody is linked to the N-terminal end of a sequence SEQ ID NO:4.

Preferably in said format, the anti-CD28 antibody light and heavy chains comprise the following first combinations of 6 CDRs :
as in Kabat:
H-CDR1: DYGVH (SEQ ID NO: 11)
H-CDR2: VIWAGGGTNYNSALMS (SEQ ID NO: 12)
H-CDR3: DKGYSYYYSMDY (SEQ ID NO: 13),
and
L-CDR1: RASESVEYYVTSLMQ (SEQ ID NO: 14)
L-CDR2: AASNVES (SEQ ID NO: 15)
L-CDR3: QQSRKVPYT (SEQ ID NO: 16).

Said first combination of CDRs is as follows in IMGT :
H-CDR1: GFSLSDYG (SEQ ID NO: 17)
H-CDR2: IWAGGGT (SEQ ID NO: 18)
H-CDR3: ARDKGYSYYYSMDY (SEQ ID NO: 19),
and
L-CDR1: ESVEYYVTSL (SEQ ID NO: 20)
L-CDR2: AA (SEQ ID NO: 21)
L-CDR3: QQSRKVPYT (SEQ ID NO: 16).

Alternatively, more preferably in said format (bivalent), the anti-CD28 antibody light and heavy chains comprise the following second combination of 6 CDRs :
as in Kabat:
H-CDR1: DYGVH (SEQ ID NO: 11)
H-CDR2: AIWAGGGTNYASSVMG (SEQ ID NO: 22)
H-CDR3: DKGYSYYYSMDY (SEQ ID NO: 13),
and
L-CDR1: RASESVEYYVTSLMA (SEQ ID NO: 23)
L-CDR2: AASNVES (SEQ ID NO: 15)
L-CDR3: QQSRKVPYT (SEQ ID NO: 16).

Said second combination of CDRs is as follows in IMGT :
H-CDR1: GFTFSDYG (SEQ ID NO: 24)
H-CDR2: IWAGGGT (SEQ ID NO: 18)
H-CDR3: ARDKGYSYYYSMDY (SEQ ID NO: 19),
and
L-CDR1: ESVEYYVTSL (SEQ ID NO: 20)
L-CDR2: AA (SEQ ID NO: 21)
L-CDR3: QQSRKVPYT (SEQ ID NO: 16).

Preferably, said bispecific protein complex comprises:
- two heavy chains of the anti-CD28 antibody linked to the N-terminal end of a soluble PD-1 protein or one of its fragments, and
- two light chains of the anti-CD28 antibody,
wherein the anti-CD28 antibody light and heavy chains comprise the following first combinations of 6 CDRs :
as in Kabat:
H-CDR1: DYGVH (SEQ ID NO: 11)
H-CDR2: VIWAGGGTNYNSALMS (SEQ ID NO: 12)
H-CDR3: DKGYSYYYSMDY (SEQ ID NO: 13),
and
L-CDR1: RASESVEYYVTSLMQ (SEQ ID NO: 14)
L-CDR2: AASNVES (SEQ ID NO: 15)
L-CDR3: QQSRKVPYT (SEQ ID NO: 16).

Preferably, said bispecific protein complex comprises:
- two heavy chains of the anti-CD28 antibody linked to the N-terminal end of a soluble PD-1 protein or one of its fragments, and
- two light chains of the anti-CD28 antibody,
wherein the anti-CD28 antibody light and heavy chains comprise the following second combinations of 6 CDRs :
as in Kabat:
H-CDR1: DYGVH (SEQ ID NO: 11)
H-CDR2: AIWAGGGTNYASSVMG (SEQ ID NO: 22)
H-CDR3: DKGYSYYYSMDY (SEQ ID NO: 13),
and
L-CDR1: RASESVEYYVTSLMA (SEQ ID NO: 23)
L-CDR2: AASNVES (SEQ ID NO: 15)
L-CDR3: QQSRKVPYT (SEQ ID NO: 16).

Preferably, said bispecific protein complex comprises:
- two heavy chains of the anti-CD28 antibody linked to the N-terminal end of a soluble PD-1 protein or one of its fragments, wherein each heavy chain is of sequence SEQ ID NO:8, and
- two light chains of the anti-CD28 antibody, wherein each light chain is of sequence SEQ ID NO:7.

Alternatively, preferably, said bispecific protein complex comprises:
- two heavy chains of the anti-CD28 antibody linked to the N-terminal end of a soluble PD-1 protein or one of its fragments, wherein each heavy chain is of sequence SEQ ID NO:9, and
- two light chains of the anti-CD28 antibody, wherein each light chain is of sequence SEQ ID NO:7.

Preferably, the bispecific protein complex of the invention is such that one of combinations a) to t) is fulfilled :
a) the anti-CD28 VL domain is of sequence SEQ ID NO :1, and
   the anti-CD28 VH domain is of sequence SEQ ID NO :2, and
   the soluble PD-1 protein fragment is of sequence SEQ ID NO :3 ;
   or
b) the anti-CD28 VL domain is of sequence SEQ ID NO :1, and
   the anti-CD28 VH domain is of sequence SEQ ID NO :2, and
   the soluble PD-1 protein fragment is of sequence SEQ ID NO :4;
   or
c) the anti-CD28 VH domain and the anti-CD28 VL domain comprise the following first combinations of 6 CDRs :
   as in Kabat:
   H-CDR1: DYGVH (SEQ ID NO: 11)
   H-CDR2: VIWAGGGTNYNSALMS (SEQ ID NO: 12)
   H-CDR3: DKGYSYYYSMDY (SEQ ID NO: 13),
   and
   L-CDR1: RASESVEYYVTSLMQ (SEQ ID NO: 14)
   L-CDR2: AASNVES (SEQ ID NO: 15)
   L-CDR3: QQSRKVPYT (SEQ ID NO: 16), and
   the soluble PD-1 protein fragment is of sequence SEQ ID NO :3 ;
   or
d) the anti-CD28 VH domain and the anti-CD28 VL domain comprise the following first combinations of 6 CDRs :
   as in Kabat:
   H-CDR1: DYGVH (SEQ ID NO: 11)
   H-CDR2: VIWAGGGTNYNSALMS (SEQ ID NO: 12)
   H-CDR3: DKGYSYYYSMDY (SEQ ID NO: 13),
   and
   L-CDR1: RASESVEYYVTSLMQ (SEQ ID NO: 14)
   L-CDR2: AASNVES (SEQ ID NO: 15)
   L-CDR3: QQSRKVPYT (SEQ ID NO: 16), and
   the soluble PD-1 protein fragment is of sequence SEQ ID NO :4;
   or
e) the anti-CD28 VH domain and the anti-CD28 VL domain comprise the following second combinations of 6 CDRs :
   as in Kabat:
   H-CDR1: DYGVH (SEQ ID NO: 11)
   H-CDR2: AIWAGGGTNYASSVMG (SEQ ID NO: 22)
   H-CDR3: DKGYSYYYSMDY (SEQ ID NO: 13),
   and
   L-CDR1: RASESVEYYVTSLMA (SEQ ID NO: 23)
   L-CDR2: AASNVES (SEQ ID NO: 15)
   L-CDR3: QQSRKVPYT (SEQ ID NO: 16), and
   the soluble PD-1 protein fragment is of sequence SEQ ID NO :3 ;
   or
f) the anti-CD28 VH domain and the anti-CD28 VL domain comprise the following second combinations of 6 CDRs :
   as in Kabat:
   H-CDR1: DYGVH (SEQ ID NO: 11)
   H-CDR2: AIWAGGGTNYASSVMG (SEQ ID NO: 22)
   H-CDR3: DKGYSYYYSMDY (SEQ ID NO: 13),
   and
   L-CDR1: RASESVEYYVTSLMA (SEQ ID NO: 23)
   L-CDR2: AASNVES (SEQ ID NO: 15)
   L-CDR3: QQSRKVPYT (SEQ ID NO: 16), and
   the soluble PD-1 protein fragment is of sequence SEQ ID NO :4;
   or
g) the anti-CD28 VH domain and the anti-CD28 VL domain are respectively of sequences SEQ ID NO:26 and SEQ ID NO:25, and the soluble PD-1 protein fragment is of sequence SEQ ID NO :3 ;
   or
h) the anti-CD28 VH domain and the anti-CD28 VL domain are respectively of sequences SEQ ID NO:26 and SEQ ID NO:25, and the soluble PD-1 protein fragment is of sequence SEQ ID NO:4;
   or
i) the anti-CD28 VH domain and the anti-CD28 VL domain comprise the following third combinations of 6 CDRs :
   as in Kabat:
   H-CDR1: GGSISSYY (SEQ ID NO:35)
   H-CDR2: IYYSGIT (SEQ ID NO:36)
   H-CDR3: ARWGVRRDYYYYGMDV (SEQ ID NO:37),
   and
   L-CDR1: QSVSSSY (SEQ ID NO:38)
   L-CDR2: GAS (SEQ ID NO:39)
   L-CDR3: QQYGSSPWT (SEQ ID NO:40), and
   the soluble PD-1 protein fragment is of sequence SEQ ID NO :3 or 4;
   or
j) the anti-CD28 VH domain and the anti-CD28 VL domain are respectively of sequences SEQ ID NO:42 and SEQ ID NO:41, and the soluble PD-1 protein fragment is of sequence SEQ ID NO :3 or 4;
k) the anti-CD28 VH domain and the anti-CD28 VL domain comprise the following fourth combinations of 6 CDRs :
   as in Kabat:
   H-CDR1 chosen from GFTFSSYG (SEQ ID NO:43) and GFTFSRNN (SEQ ID NO:44)
   H-CDR2 chosen from ISYAGNNK (SEQ ID NO:45) and ISSNGGRT (SEQ ID NO:46)
   H-CDR3 chosen from AKDSYYDFLTDPDVLDI (SEQ ID NO:47) and TRDDELLSFDY (SEQ ID NO:48),
   and
   L-CDR1: QSISSY (SEQ ID NO:49)
   L-CDR2: AAS (SEQ ID NO:50)
   L-CDR3: QQSYSTPPIT (SEQ ID NO:51), and
   the soluble PD-1 protein fragment is of sequence SEQ ID NO :3 or 4;
   or
l) the anti-CD28 VH domain and the anti-CD28 VL domain comprise the following fifth combinations of 6 CDRs :
   as in Kabat:
   H-CDR1: GFTFSRNN (SEQ ID NO:44)
   H-CDR2: ISSNGGRT (SEQ ID NO:46)
   H-CDR3: TRDDELLSFDY (SEQ ID NO:48),
   and
   L-CDR1: QSISSY (SEQ ID NO:49)
   L-CDR2: AAS (SEQ ID NO:50)
   L-CDR3: QQSYSTPPIT (SEQ ID NO:51), and
   the soluble PD-1 protein fragment is of sequence SEQ ID NO :3 or 4;
   or
m) the anti-CD28 VH domain and the anti-CD28 VL domain are respectively of sequences SEQ ID NO:56 and SEQ ID NO:55, and the soluble PD-1 protein fragment is of sequence SEQ ID NO :3 or 4;
   or
n) the anti-CD28 VH domain and the anti-CD28 VL domain comprise the following sixth combinations of 6 CDRs :
   as in Kabat:
   H-CDR1: GYTFTSYY (SEQ ID NO:57)
   H-CDR2: IYPGNVNT (SEQ ID NO:58)
   H-CDR3: TRSHYGLDWNFDV (SEQ ID NO:59),
   and
   L-CDR1: QNIYVW (SEQ ID NO:60)
   L-CDR2: KAS (SEQ ID NO:61)
   L-CDR3: QQGQTYPY (SEQ ID NO:62), and
   the soluble PD-1 protein fragment is of sequence SEQ ID NO :3 or 4;
   or
o) the anti-CD28 VH domain and the anti-CD28 VL domain are respectively of sequences SEQ ID NO:64 and SEQ ID NO:63, and the soluble PD-1 protein fragment is of sequence SEQ ID NO:3 or 4;
p) the anti-CD28 VH domain and the anti-CD28 VL domain comprise the following seventh combinations of 6 CDRs :
   as in Kabat:
   H-CDR1: SYAMS (SEQ ID NO:65)
   H-CDR2: TISGSGDSTYYADSVKG (SEQ ID NO:66)
   H-CDR3: SGPGLRQVGFDY (SEQ ID NO:67)
   L-CDR1 :RASQSISSYLN (SEQ ID NO:68)
   L-CDR2: AASSLQS (SEQ ID NO:69)
   L-CDR3: QQSYSTPFT (SEQ ID NO:70), and
   the soluble PD-1 protein fragment is of sequence SEQ ID NO :3 or 4;
   or
q) the anti-CD28 VH domain and the anti-CD28 VL domain comprise the following seventh combinations of 6 CDRs :
   as in Kabat:
   H-CDR1: SYAMS (SEQ ID NO:65)
   H-CDR2: TISGSGDSTYYADSVKG (SEQ ID NO:66)
   H-CDR3: SGPGLRQVGFDY (SEQ ID NO:67)
   L-CDR1:RASQSISSYLN (SEQ ID NO:68)
   L-CDR2: AASSLQS (SEQ ID NO:69)
   L-CDR3: QQVYSTPFT (SEQ ID NO:71), and
   the soluble PD-1 protein fragment is of sequence SEQ ID NO :3 or 4;
   or
r) the anti-CD28 VH domain and the anti-CD28 VL domain comprise the following seventh combinations of 6 CDRs :
   as in Kabat:
   H-CDR1: SYYMS (SEQ ID NO:72)
   H-CDR2: TISGSGDSTYYADSVKG (SEQ ID NO:66)
   H-CDR3: SGPGLRQVGFDY (SEQ ID NO:67)
   L-CDR1:RASQSISSYLN (SEQ ID NO:68)
   L-CDR2: AASSLQS (SEQ ID NO:69)
   L-CDR3: QQVYSTPFT (SEQ ID NO:71), and
   the soluble PD-1 protein fragment is of sequence SEQ ID NO :3 or 4;
   or
s) the anti-CD28 VH domain and the anti-CD28 VL domain comprise the following seventh combinations of 6 CDRs :
   as in Kabat:
   H-CDR1: SYYMS (SEQ ID NO:72)
   H-CDR2: TISESGDSTYYADSVKG (SEQ ID NO:73)
   H-CDR3: SGPGLRQVGFDY (SEQ ID NO:67)
   L-CDR1:RASQSISSYLN (SEQ ID NO:68)
   L-CDR2: AASSLQS (SEQ ID NO:69)
   L-CDR3: QQSYSTPFT (SEQ ID NO:70), and
   the soluble PD-1 protein fragment is of sequence SEQ ID NO :3 or 4;
   or
t) the anti-CD28 VH domain and the anti-CD28 VL domain comprise the following seventh combinations of 6 CDRs :
   as in Kabat:
   H-CDR1: SYYMS (SEQ ID NO:72)
   H-CDR2: TISESGDSTYYADSVKG (SEQ ID NO:73)
   H-CDR3: SGPGLRQVGFDY (SEQ ID NO:67)
   L-CDR1:RASQSISSYLN (SEQ ID NO:68)
   L-CDR2: AASSLQS (SEQ ID NO:69)
   L-CDR3: QQVYSTPFT (SEQ ID NO:71), and
   the soluble PD-1 protein fragment is of sequence SEQ ID NO :3 or 4.

### Therapeutic uses

The present invention also relates to the use of a bispecific protein complex of the invention as a medicament.

The present invention also relates to the use of a bispecific protein complex of the invention for treating a cancer. Preferably the cancer is a solid or a liquid tumor.

It is also described a method for treating a cancer in a subject in need thereof comprising a step of administering to said subject a therapeutically effective amount of a bispecific protein complex according the invention.

Cancer refers to tumors. The tumors to be treated include primary tumors and metastatic tumors, as well as refractory tumors. Refractory tumors include tumors that fail to respond or are resistant to treatment with chemotherapeutic agents alone, antibodies alone, radiation alone or combinations thereof. Refractory tumors also encompass tumors that appear to be inhibited by treatment with such agents, but recur up to five years, sometimes up to ten years or longer after treatment is discontinued.

Examples of cancers that may be treated by the bispecific protein complex of the invention include, but are not limited to, cancer cells from the bladder, blood, bone, bone marrow, brain, breast, colon, esophagus, gastrointestine, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous; adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; and roblastoma, malignant; Sertoli cell carcinoma; leydig cell tumor, malignant; lipid cell tumor, malignant; paraganglioma, malignant; extra- mammary paraganglioma, malignant; pheochromocytoma; glomangio sarcoma; malignant melanoma; amelanotic melanoma; superficial spreading melanoma; malig melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangio sarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non- Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia.

As used herein, the term "treatment" or "treat" refer to both prophylactic or preventive treatment as well as curative or disease modifying treatment, including treatment of subjects at risk of contracting the disease or suspected to have contracted the disease as well as subjects who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment.

By "therapeutic regimen" is meant the pattern of treatment of an illness, e.g., the pattern of dosing used during therapy. A therapeutic regimen may include an induction regimen and a maintenance regimen.

The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both.

The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., disease manifestation, etc.]).

By a "therapeutically effective amount" is meant a sufficient amount of the bispecific protein complex of the invention to treat the disease (e.g. cancer) at a reasonable benefit/risk ratio applicable to any medical treatment. It will be understood that the total daily usage of the bispecific protein complex of the present invention will be decided by the attending physician within the scope of sound medical judgment. The specific therapeutically effective dose level for any particular patient will depend upon a variety of factors including the age, body weight, general health, sex and diet of the patient; the time of administration, route of administration, and rate of excretion of the specific compound employed; the duration of the treatment; drugs used in combination or coincidental with the bispecific protein complex employed; and like factors well known in the medical arts. For example, it is well known within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, typically from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day. The bispecific protein complex of the present invention may be combined with pharmaceutically acceptable excipients, and optionally sustained-release matrices, such as biodegradable polymers, to form pharmaceutical compositions.

"Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Typically, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The bispecific protein complex can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile- filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

### Preparation methods of the bispecific protein complex

The bispecific protein complex of the invention may be prepared according to conventional methods known in the art.

For example, a method for producing a bispecific protein complex of the invention may comprise the following steps :
preparing a cDNA expression vector encoding the anti-CD28 heavy chain sequence linked to the soluble PD-1 protein, a cDNA expression vector encoding the hinge-CH2-CH3 domain linked to the soluble PD-1 protein and a cDNA expression vector encoding the anti-CD28 light chain ;
co-transfecting the three cDNA obtained in the previous steps in host cells, such as CHO cells ;
incubating said transfected host cells ; and then
collecting the obtained protein complexes, for example by using affinity chromatography and/or size-exclusion chromatography and/or hydrophobic interaction chromatography and/or ion exchange chromatography.

The bispecific protein complex of the invention may be prepared as described in the examples.

The invention will be further illustrated by the following figures and examples. However, these examples and figures should not be interpreted in any way as limiting the scope of the present invention.

### FIGURES LEGENDS

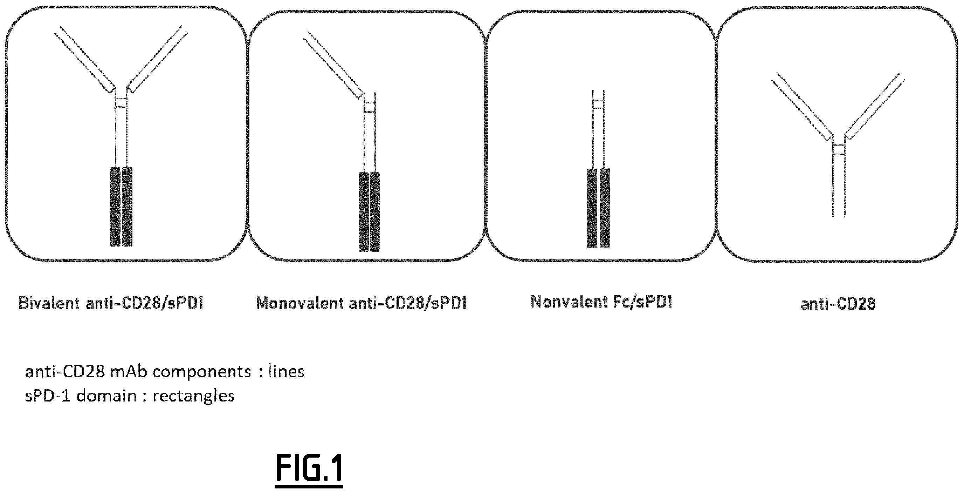

**Figure 1** is a schematic representation of the molecules with anti-CD28 mAb components shown as lines and sPD-1 domain depicted as rectangles.

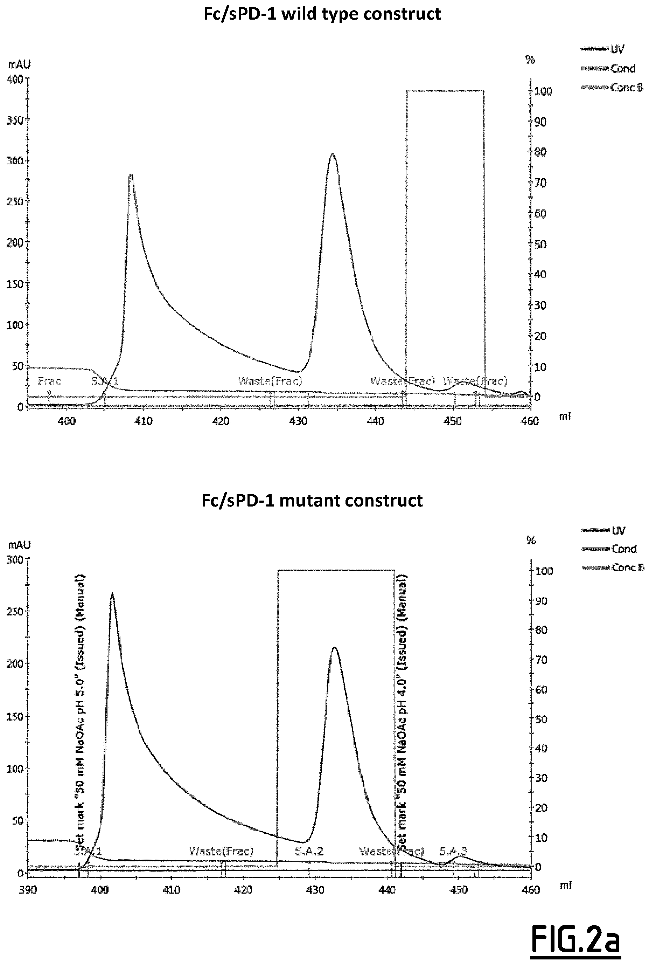

**Figure 2a** is a graph depicting the purification of anti-CD28/sPD-1 molecules of the invention by CaptureSelect CH1-XL affinity chromatography from the supernatant of transfected CHO cells. Proteins were eluted from the column using 50 mM sodium acetate buffer at either pH 5.0 (enriched monovalent fraction) or pH 4.5 (enriched bivalent fraction). The left panel depicts the Fc/sPD-1 wild type construct. The right panel depicts the Fc/sPD-1 mutant construct.

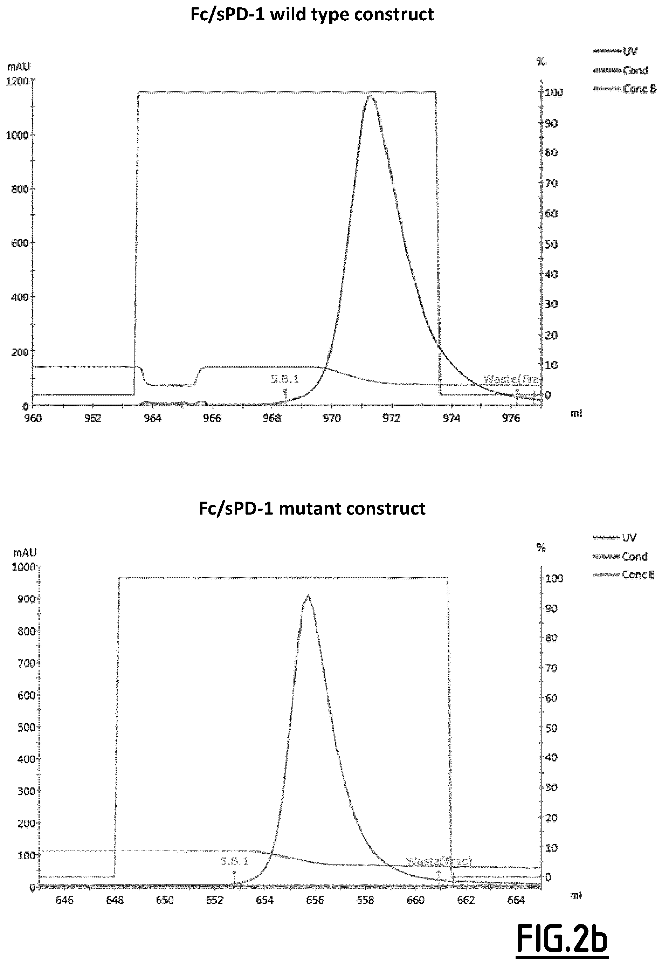

**Figure 2b** is a graph depicting the purification of Fc/sPD-1 molecules by protein A affinity chromatography from the flow through of CaptureSelect CH1-XL chromatography. Proteins were eluted from the column using 100 mM sodium citrate buffer at pH 3.3. The left panel depicts the Fc/sPD-1 wild type construct. The right panel depicts the Fc/sPD-1 mutant construct.

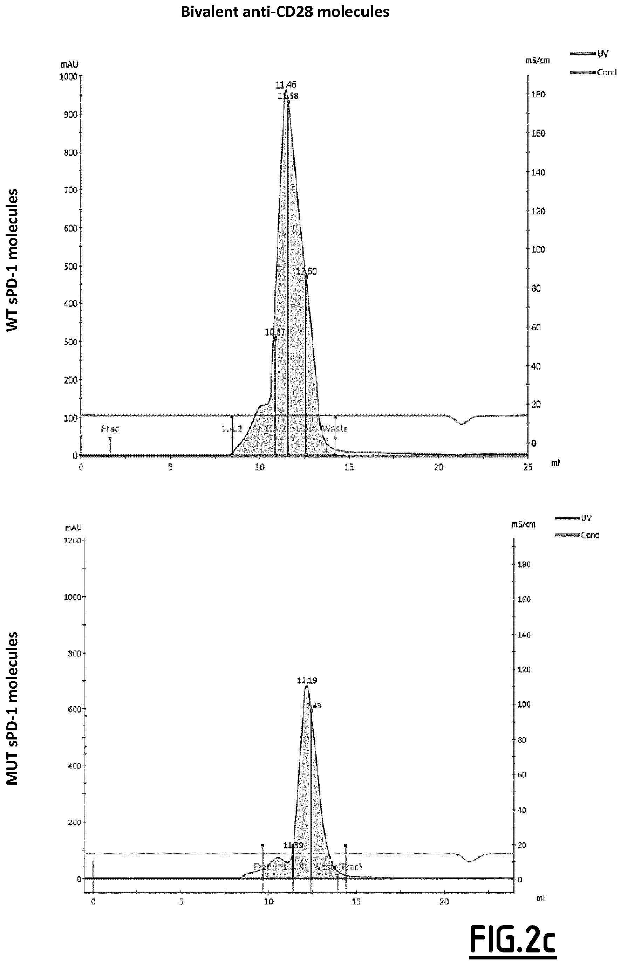

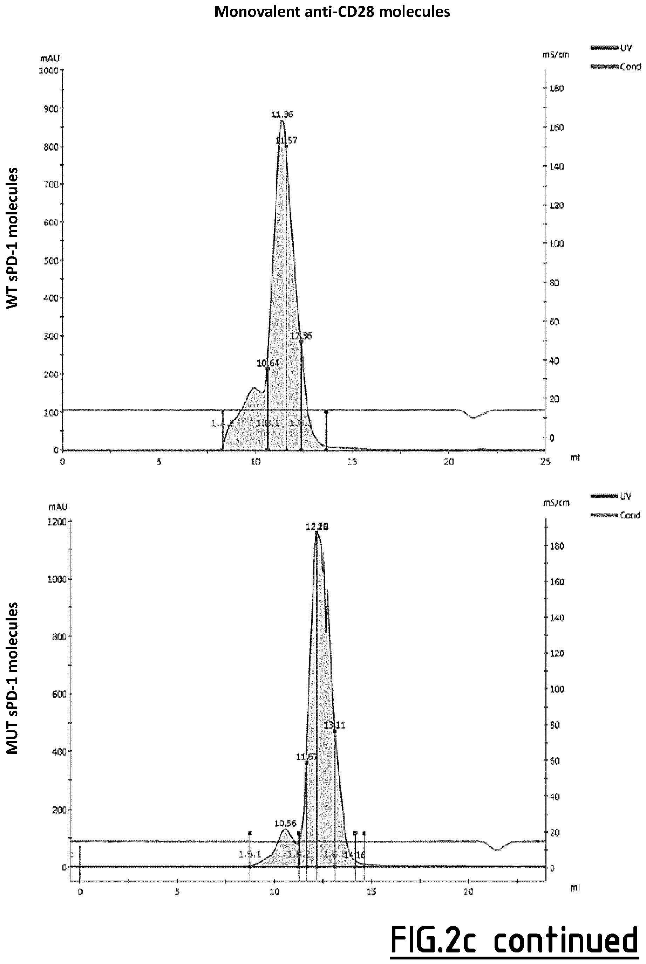

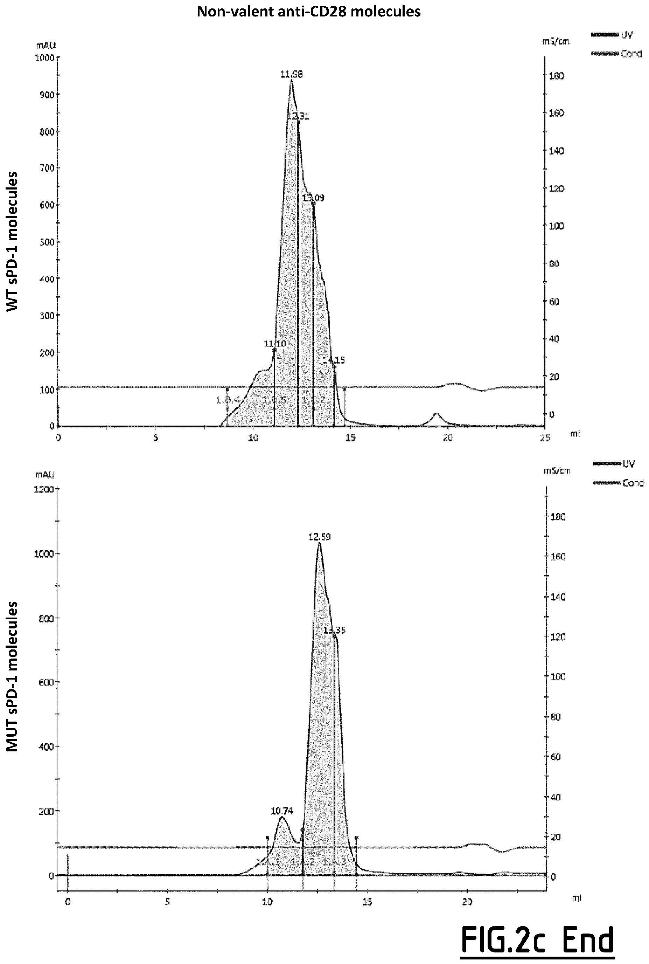

**Figure 2c** is a graph depicting "Polishing" purification by size exclusion chromatography. Eluated from previous affinity chromatography steps were loaded on a Superdex 200 10/300 GL column and eluted using 25 mM Histidine, 125 mM NaCl, 0.02% Polysorbate 80. Fractions of interest were pooled based on their purity profile by non-reducing SDS-PAGE against their expected size. Left panels: Bivalent anti-CD28 molecules. Middle panels: Monovalent anti-CD28 molecules. Right panels: Non-valent anti-CD28 molecules. Upper panels: Wild type sPD-1 molecules. Lower panels: Mutated sPD-1 molecules.

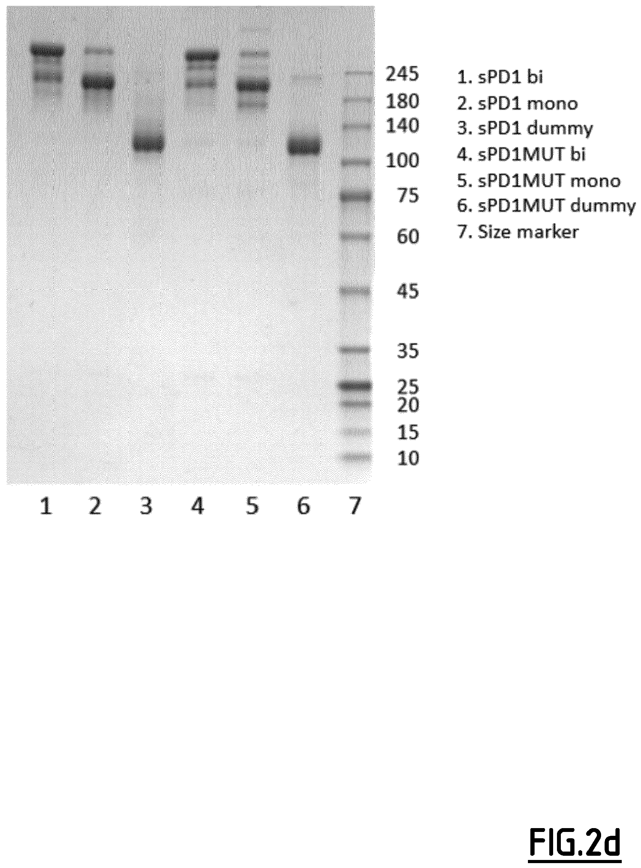

**Figure 2d** is an analysis of pooled size-exclusion chromatography fractions by non-reduced SDS-PAGE Coomassie staining. 0.8 ug of protein was loaded on 4-15% gradient SDS-PAGE. Samples were heated at 70°C for 10 minutes before loading.

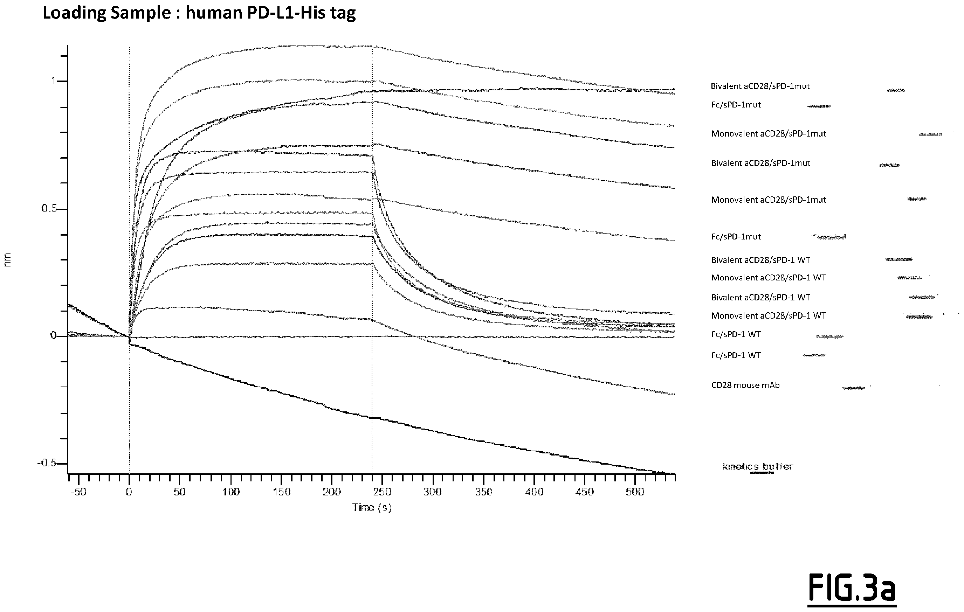

**Figure 3a** is a graph of Octet binding studies on sPD-L1. Recombinant human PD-L1-His tag (ACROBiosystems, #PD1-H5229) was loaded to His-sensor (HIS1K (Anti-Penta-His)) at 10 µg/ml and the binding of each construct was evaluated by Octet RED96e.

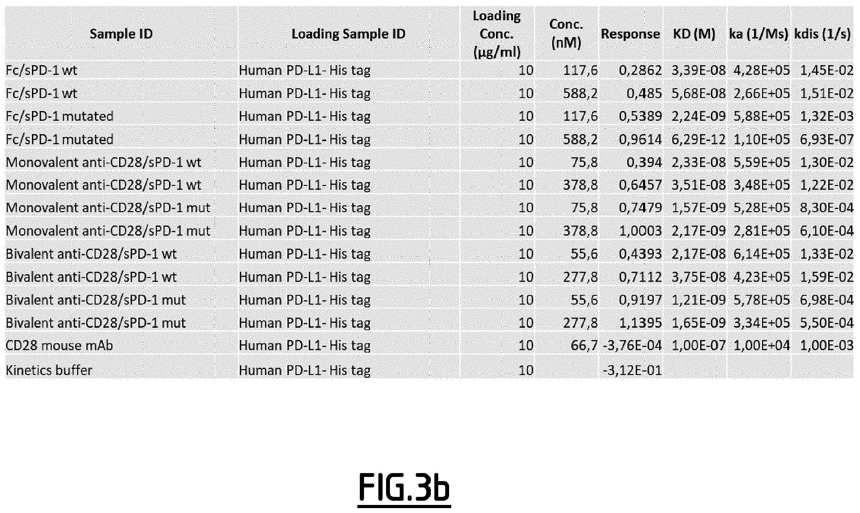

**Figure 3b** is a table of affinity and kinetics constants for each construct on human PD-L1-His tag protein by Octet RED96e.

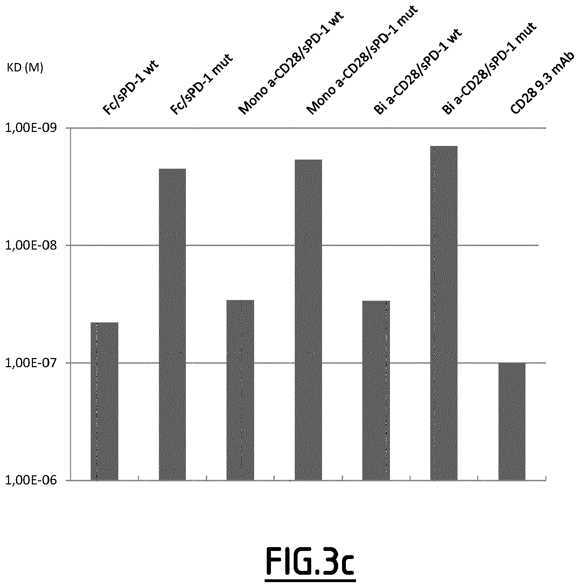

**Figure 3c** is a graph depicting the KD values of anti-CD28/sPD-1 constructs on human PD-L1-His tag protein by Octet RED96e. The equilibrium dissociation constant (KD) is calculated as the ratio k_{off}/kₒₙ (with k_{off}: dissociation rates, and kₒₙ: association rates). Monoclonal anti-CD28 (9.3) mAb (GeneTex, # GTX14664) was used as a negative control.

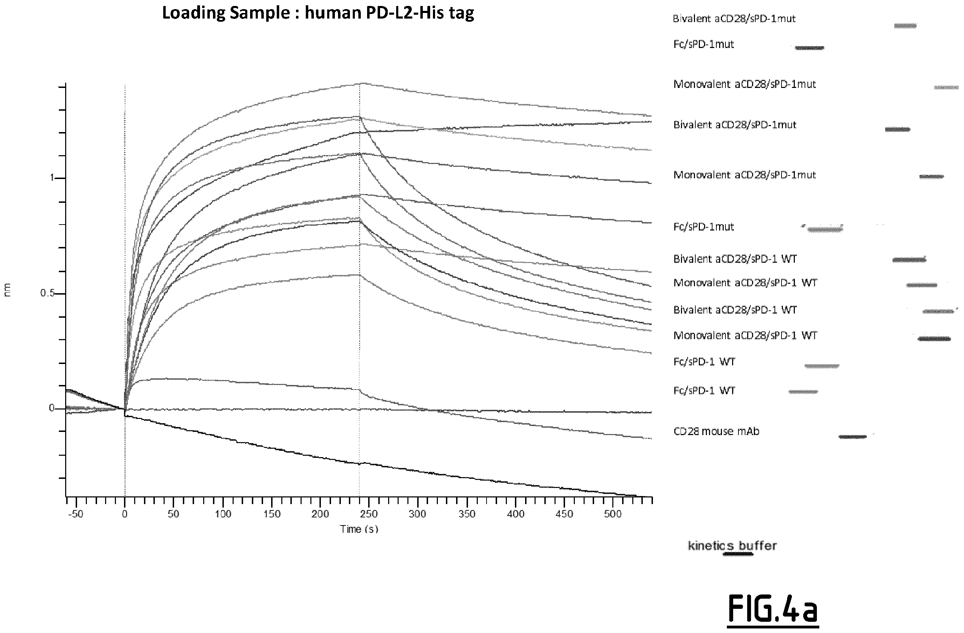

**Figure 4a** is a graph of Octet binding studies on sPD-L2. Recombinant human PD-L2-His tag (ACROBiosystems, #PD2-H5220) was loaded to His-sensor (HIS1K (Anti-Penta-His)) at 10 µg/ml and the binding of each construct was evaluated by Octet RED96e.

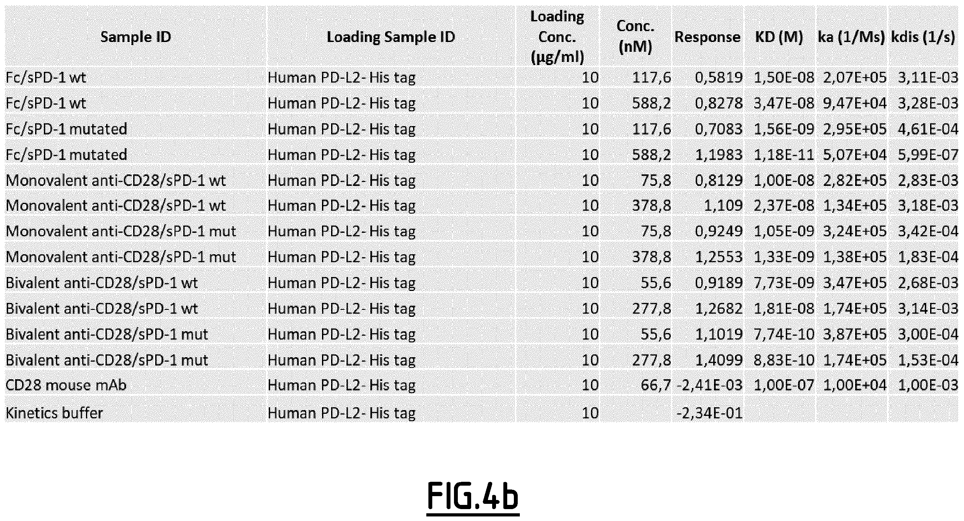

**Figure 4b** is a table of affinity and kinetics constants for each construct on human PD-L2-His tag protein by Octet RED96e.

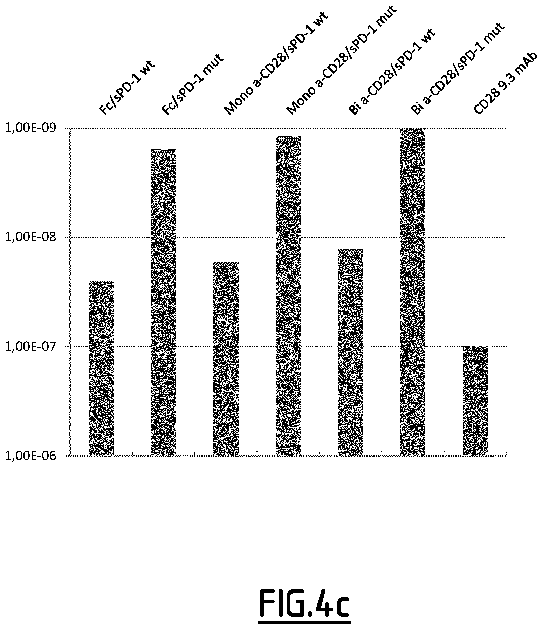

**Figure 4c** is a graph depicting the KD values of anti-CD28/sPD-1 constructs on human PD-L2-His tag protein by Octet RED96e. The equilibrium dissociation constant (KD) is calculated as the ratio k_{off}/kₒₙ (with k_{off}: dissociation rates, and kₒₙ: association rates). Monoclonal anti-CD28 (9.3) mAb (GeneTex, # GTX14664) was used as a negative control.

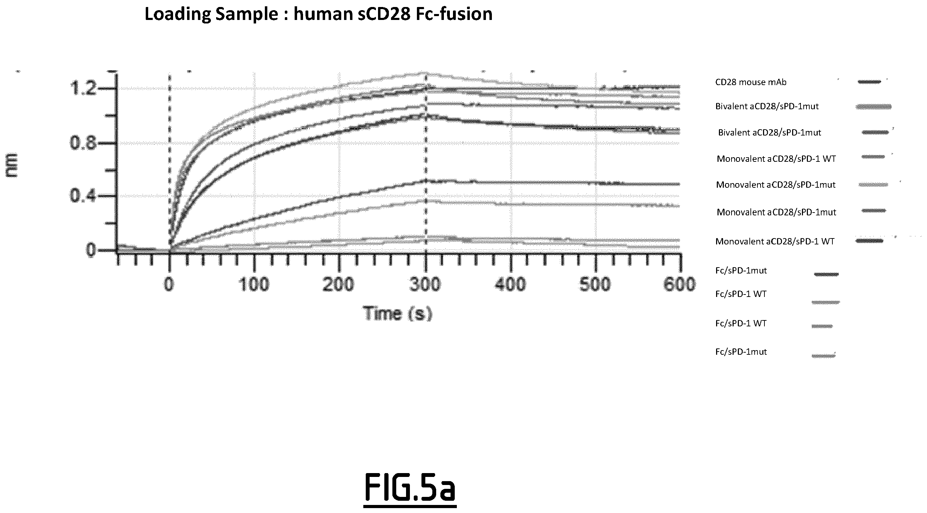

**Figure 5a** is a graph of Octet binding studies on sCD28. Recombinant human sCD28 Fc-fusion protein (PeproTech, #310-34) was loaded to protA sensor at 2.5 µg/ml and the binding of each construct was evaluated by Octet RED96e.

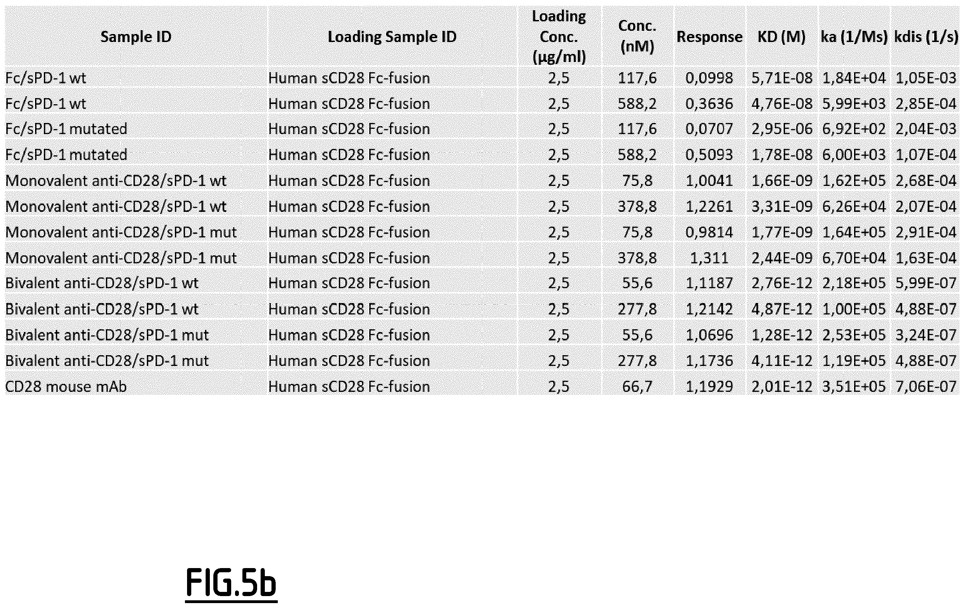

**Figure 5b** is a table of affinity and kinetics constants for each construct on human sCD28 Fc-fusion protein by Octet RED96e.

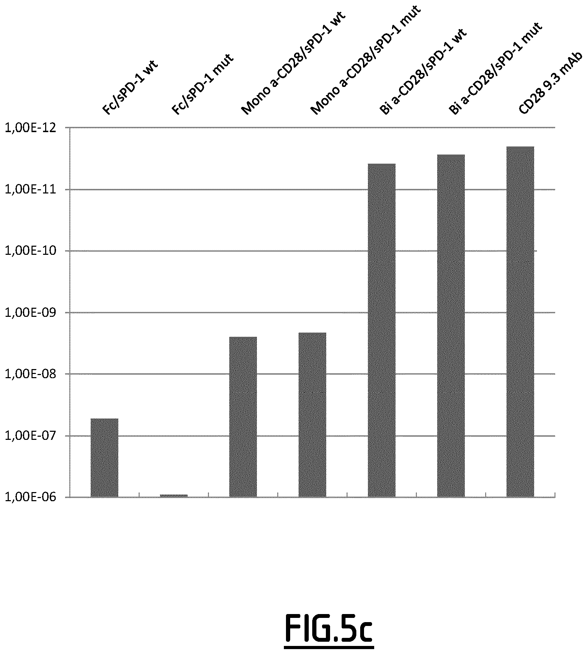

**Figure 5c** is a graph depicting the KD values of anti-CD28/sPD-1 constructs on human sCD28 Fc-fusion protein by Octet RED96e. The equilibrium dissociation constant (KD) is calculated as the ratio k_{off}/kₒₙ (with k_{off}: dissociation rates, and kₒₙ: association rates). Monoclonal anti-CD28 (9.3) mAb (GeneTex, # GTX14664) was used as a positive control.

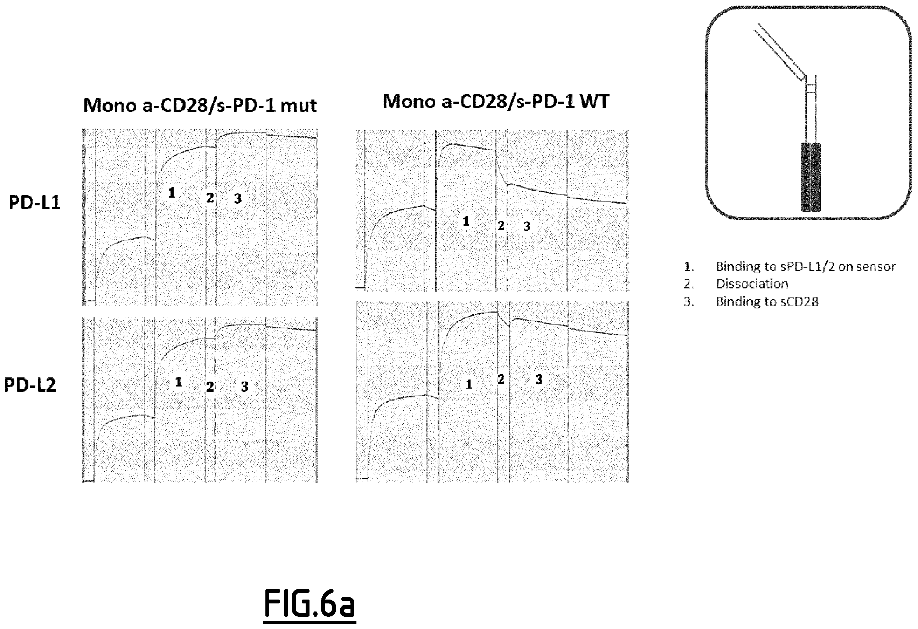

**Figure 6a** is a graph showing co-binding raw data of monovalent anti-CD28 molecules of the invention by Octet. Association of **Monovalent anti-CD28/sPD-1 Mutated structure of the invention** ("Mono Mut") to PD-L1 (upper left figure) and to PD-L2 (bottom left figure) is visible in step 1, Low dissociation of Mono from PD-L1/PD-L2 is visible in step 2. Undeniable association of sCD28-Fc to Mono Mut is in step 3. Association of **Monovalent anti-CD28/sPD-1 Wild type structure of the invention** ("Mono") to PD-L1 (upper right figure) and to PD-L2 (bottom right figure) in step 1, high dissociation of Mono from PD-L1/PD-L2 is visible in step 2. In sCD28-Fc association step (step 3), dissociation slows down probably due to binding of sCD28 to remaining Mono molecules on PD-L1/PD-L2.

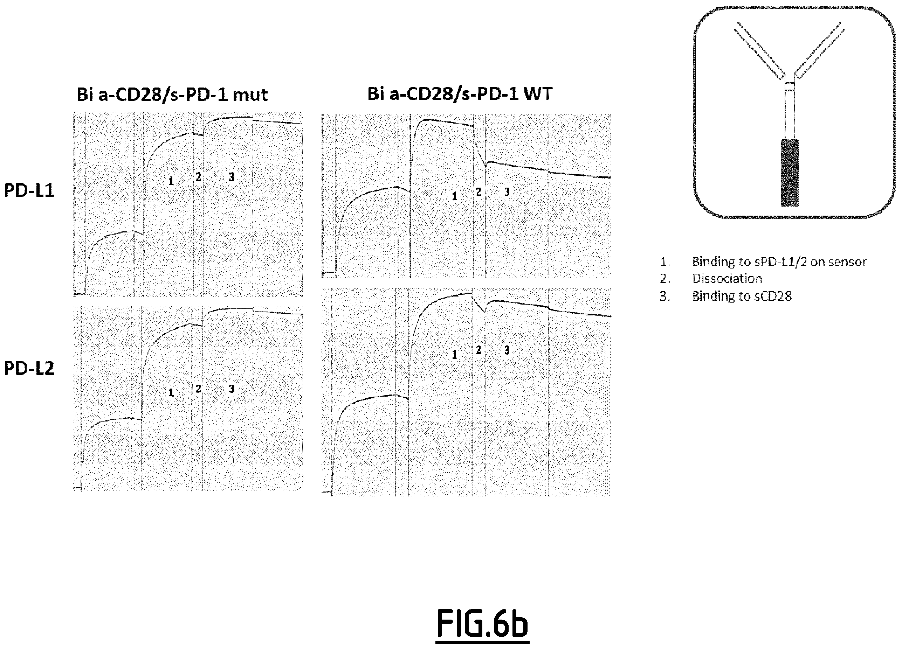

**Figure 6b** is a graph showing co-binding raw data of bivalent anti-CD28 molecules by Octet. Association of **Bivalent anti-CD28/sPD-1 Mutated structure of the invention** ("Bi Mut") to PD-L1 (upper left figure) and to PD-L2 (bottom left figure) in step 1, Low dissociation of Bi Mut from PD-L1/PD-L2 is visible in step 2. Undeniable association of sCD28-Fc to Bi Mut is in step 3. Association of **Bivalent anti-CD28/S-PD-1 Wild type structure of the invention** ("Bi") to PD-L1 (upper right figure) and to PD-L2 (bottom right figure) in step 1, high dissociation of Bi from PD-L1/PD-L2 is visible in step 2. In sCD28-Fc association step (step 3), dissociation slows down probably due to binding of sCD28 to remaining Bi molecules on PD-L1/PD-L2.

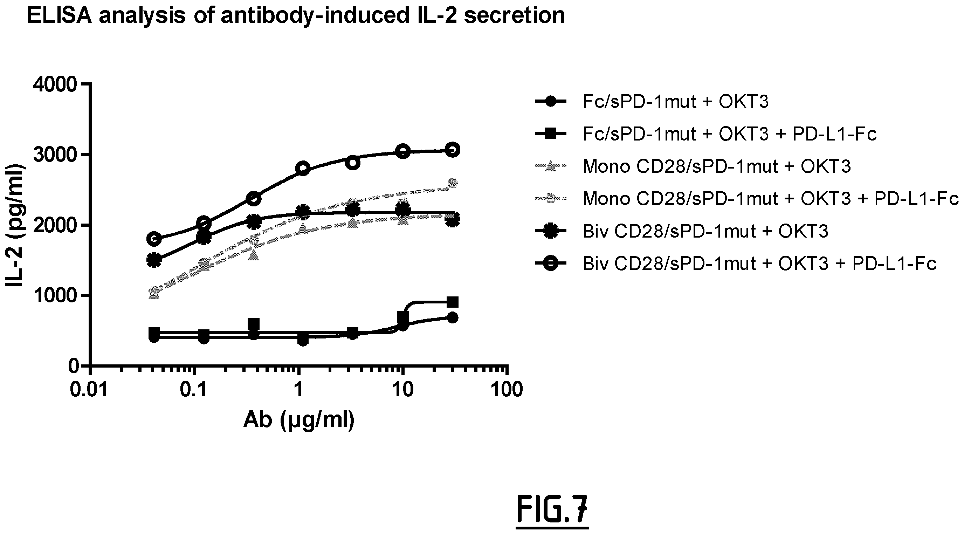

**Figure 7** is a graph showing IL-2 secretion by Jurkat T cells in response to anti-CD28/sPD-1 constructs. Jurkat T cells were activated with suboptimal PMA (15 ng/ml) and preincubated with anti-PD-1 mAb (saturating concentration 10 ug/ml) to prevent sPD-L1-Fc from engaging PD-1. Then, Jurkat T cells were stimulated by serial dilutions of anti-CD28 - sPD-1 mutated constructs, either alone, or in combination with anti-CD3 (OKT3) antibody (1 ug/ml), or cross-linking reagent (IgG or sPD-L1-Fc (PeproTech, #310-35), 5 µg/ml). Culture supernatants were collected 48h later, and IL-2 secretion was analyzed by ELISA.

### EXAMPLES

### Molecule Design and Production

Monoclonal antibody 9.3 *(*Hansen JA et al, Immunogenetics, 1980*)* was used as the CD28 binding moiety, either in bivalent (i.e. 2 Fabs) or monovalent (i.e. one Fab) modality. For binding to PD-1 ligands PD-L1 and PD-L2, the extracellular domain of PD-1 was used (sPD-1). The sPD-1 domain was fused to the C-terminus of the Fc (i.e. following the CH3 domain) via a flexible glycine/serine linker (3 x GGGGS). As controls, molecules without CD28 binding Fab domains were generated (i.e. Fc/sPD-1) as well as the 9.3 mAb without C-terminal fusion to sPD-1 (the latter being purchased from a commercial source, (GeneTex, # GTX14664)). A diagram of the molecules is shown in Figure 1.

To produce the sPD1 fusion constructs, a cDNA expression vector encoding the 9.3 heavy chain sequence fused to the sPD-1 domain was co-transfected in CHO cells with a cDNA expression vector encoding the Fc domain alone fused to the sPD-1 domain as well as a cDNA expression vector encoding the 9.3 light chain. This resulted in a mixture of products in the CHO supernatant with a ratio of 25% bivalent, 50% monovalent, 25% non-valent molecules based on random chain assembly. Two different sPD-1 domains were evaluated, one corresponding to the wild type sequence and one corresponding to a mutated version with increased affinity for the PD-1 ligands PD-L1 and PD-L2 (containing mutations G124S, K131Y and A132I). Amino acid sequences are described in the table above, see SEQ ID NO :30-34.

The different products were purified using CaptureSelect CH1-XL affinity chromatography. This chromatography resin uses a stable ligand that binds with high specificity and affinity to the CH1 domain of the antibody heavy chain. CHO supernatant was loaded onto the affinity column. Bivalent and monovalent products were enriched from each other via pH step elutions at pH 5.0 (enrichment of monovalent) and pH 4.5 (enrichment of bivalent) elutions (Figure 2a). Non-valent Fc/sPD-1 molecule was not retained by the column and hence was recovered from the flow through/wash fractions by protein A affinity which has high specificity and affinity for the Fc portion of antibodies (Figure 2b). The molecules were further purified by size-exclusion chromatography (Figure 2c). Final purified material was analysed by non-reducing SDS-PAGE to control for purity (Figure 2d).

### Anti-CD28/sPD-1 Antibody Fusion Constructs bind PD-1 ligands

Anti-CD28/sPD-1 antibody fusion constructs were assessed for their ability to bind PD-1 ligands, PD-L1 and PD-L2 by Octet RED96e. Recombinant human PD-L1-His tag (ACROBiosystems, #PD1-H5229) or recombinant human PD-L2-His tag (ACROBiosystems, #PD2-H5220) were loaded to His-sensor (HIS1K (Anti-Penta-His)) at 10 µg/ml.

Figure 3a shows the results of binding for each construct to human PD-L1-His tag by Octet. Affinity and kinetics constants are listed in Figure 3b. Figure 3c is a summary of affinity (measured as Kd, M) for each construct on human PD-L1. Figure 4a shows the results of binding for each construct to human PD-L2-His tag by Octet. Affinity and kinetics constants are listed in the Figure 4b. Figure 4c is a summary of affinity (measured as Kd, M) for each construct on human PD-L2.

As shown in Figures 3 and 4, mutated sPD-1 molecules showed increased affinity to PD-L1 and PD-L2 (~ 1.10⁻⁹ M) compared to wild-type sPD-1 molecules (~ 1.10⁻⁸ M).

### Anti-CD28/sPD-1 Antibody Fusion Constructs bind CD28

Anti-CD28/sPD-1 antibody fusion constructs were assessed for their ability to bind CD28 by Octet RED96e. Recombinant human sCD28 Fc-fusion protein (PeproTech, #310-34) was loaded to protA sensor at 2.5 µg/ml.

Figure 5a shows the results of binding for each construct to human sCD28 Fc-fusion protein by Octet. Affinity and kinetics constants are listed in the Figure 5b. Figure 5c is a summary of affinity (measured as Kd, M) for each construct on human CD28.

As shown in Figure 5, bivalent anti-CD28 molecules showed increased affinity (~ 1.10⁻¹² M) compared to monovalent anti-CD28 molecules (~ 1.10⁻⁹ M). However, anti-CD28 -sPD-1 antibody fusion constructs retain ability to bind soluble CD28 in the monovalent format.

### Anti-CD28/sPD-1 Antibody Fusion Constructs concomitantly bind CD28 and a PD-1 ligand

Anti-CD28/sPD-1 antibody fusion constructs were assessed for their ability to simultaneously bind CD28 and PD-L1 or PD-L2 by a multilayer Octet RED96e.

First, His-sensors were used to immobilize human PD-L1-His tag protein and human PD-L2-His tag proteins (loaded at 20 ug/ml), separately. Residual free binding sites of protein A were next blocked with polyclonal rabbit antibodies. Next, binding of antibody samples (at 50 ug/ml excl. CD28 9.3 mAb-10 µg/ml, in kinetics buffer) to PD-L1-His/PD-L2-His were monitored. Then, association of sCD28 Fc-fusion protein (20 ug/ml) to the antibodies was registered. Kinetics data were analysed.

Controls (isotype control and CD28 9.3 mAb) did not bind to PD-L1/PD-L2 (loaded to sensors).

Fc/sPD-1 wild type and Fc/sPD-1 mutated did bind to PD-L1/PD-L2 but had no binding activity towards sCD28-Fc.

Monovalent anti-CD28/sPD-1 wt and monovalent anti-CD28/sPD-1 mut did bind to PD-L1/PD-L2 and had a binding activity towards sCD28-Fc, as shown in Figure 6a. Bivalent anti-CD28/sPD-1 wt and bivalent anti-CD28/sPD-1 mutated did bind to PD-L1/PD-L2 and had a binding activity towards sCD28-Fc, as shown in Figure 6b. These results show that anti-CD28 -sPD-1 Antibody Fusion Constructs are capable of simultaneous binding to both CD28 and PD-L1 or PD-L2.

### Anti-CD28/sPD-1 Antibody Fusion Constructs, in their monovalent and bivalent format, activate IL-2 secretion by Jurkat T cells

Anti-CD28/sPD-1 mutated antibody fusion constructs were assessed for their ability to activate IL-2 secretion by Jurkat T cells.

Jurkat T cells were activated with a suboptimal PMA concentration (15 ng/ml) and preincubated with anti-PD-1 mAb (saturating concentration 10 ug/ml) to prevent sPD-L1-Fc from engaging PD-1. Then, Jurkat T cells were stimulated by serial dilutions of anti-CD28 - sPD-1 mutated constructs, in combination with anti-CD3 (OKT3) antibody (1 µg/ml), or cross-linking reagent (IgG or sPD-L1 -Fc, 5 ug/ml). Culture supernatants were collected 48h later, and IL-2 secretion was analyzed by ELISA.

Figure 7

## Claims

1. A bispecific protein complex having the formula A:B-C-X:(D)ₘ-C'-Y wherein:
A is chosen from VL-(CL)ₙ, VL-(CH1)ₙ and VH, wherein
VL is a light chain variable region of an antibody directed against CD28,
CL is a light chain constant region of an antibody,
CH1 is the first domain of a heavy chain constant region of an antibody, and
VH is the heavy chain variable region of an antibody directed against CD28 which forms with VL a binding site to CD28, and
n is an integer which is 0 or 1,B-C-X is a first fusion protein, wherein :
B is chosen from VH-(CH1)ₙ, VH-(CL)ₙ and VL, wherein VH, CH1, CL, VL and n are as defined for A,
C is the hinge-CH2-CH3 domain of an antibody,
X is a soluble PD-1 protein or one of its fragments, and
the C-terminal end of the CH3 domain of C is linked to the N-terminal end of X ;
with the proviso than when A is VH, then B is VL, and
with the proviso than when n is 1, then A is VL-CL and B is VH-CH1, or A is VL-CH1 and B is VH-CL,
(D)ₘ-C'-Y is a second fusion protein, wherein :
D is a protein or a protein dimer,
m is an integer which is 0 or 1,
C' is a amino acid sequence which shows at least 80% identity, preferably at least 85% identity, preferably at least 90% identity, preferably at least 95% identity with C ;
Y is a soluble PD-1 protein or one of its fragments, and Y is identical to or different from X, and
the C-terminal end of the CH3 domain of C' is linked to the N-terminal end of Y ; : is a binding interaction respectively between A and B, and between C of the first fusion protein and C' of the second fusion protein.

2. The bispecific protein complex of claim 1, wherein X and Y are identical, and preferably VL, VH, CH1 and hinge-CH2-CH3 domain belong to one single antibody directed against CD28.

3. The bispecific protein complex of claim 1 or 2, wherein the binding interactions respectively between A and B, and between C of the first fusion protein and C' of the second fusion protein, are chosen from disulfide bonds and linkers; preferably the binding interactions respectively between A and B, and between C of the first fusion protein and C' of the second fusion protein, are disulfide bonds.

4. The bispecific protein complex of any one of claims 1 to 3, wherein m is 0, A is VL-CL, B is VH-CH1, the binding interactions are disulfide bonds, and preferably C' is identical to C.

5. The bispecific protein complex of any one of claims 1 to 3, wherein m is 1 and D is either an scFv that binds CD28, or a protein heterodimer that binds CD28, preferably a Fab fragment that binds CD28.

6. A bispecific protein complex comprising an anti-CD28 antibody, wherein each C-terminal end of the CH3 domain of the heavy chain of the antibody is linked to the N-terminal end of a soluble PD-1 protein or one of its fragments.

7. The bispecific protein complex of any one of claims 1 to 6, wherein the soluble PD-1 protein or one of its fragments is chosen from wild-type soluble PD-1 proteins and their fragments, and soluble PD-1 proteins comprising at least one mutation and their fragments; preferably it is chosen from the human wild-type soluble PD-1 protein and its fragments of 140 to 145 amino acids, and human soluble PD-1 proteins comprising at least one mutation as compared to the wild-type and their fragments of 140 to 145 amino acids.

8. The bispecific protein complex of claim 7, wherein the mutation increases the affinity of PD-1 to PD-L1 and/or PD-L2 as compared to the wild-type protein.

9. The bispecific protein complex of claim 7 or 8, wherein the at least one mutation is a substitution, which is preferably selected from G124S, K131Y, A132I, A132V, A132L, V64H, N66I, N66V, Y68H, M70E, M70I, N74G, T76P, K78T, S87G, S87W, C93A, N116S, L122V, A125V, S127V, S127A, K135M, A140V and their combinations,
wherein the amino acid numbering is the one of human wild-type full-length PD-1 protein, and
provided that when at least two mutations are present, they do not occur on the same position.

10. The bispecific protein complex of any one of claims 1 to 9, wherein the soluble PD-1 protein comprises the combinations of mutations G124S/K131Y/A132I, wherein the amino acid numbering is the one of human wild-type full-length PD-1 protein.

11. The bispecific protein complex of any one of claims 1 to 10, wherein the anti-CD28 antibody light and heavy chains comprise the following first combination of 6 CDRs :
as in Kabat:
H-CDR1: DYGVH (SEQ ID NO: 11)
H-CDR2: VIWAGGGTNYNSALMS (SEQ ID NO: 12)
H-CDR3: DKGYSYYYSMDY (SEQ ID NO: 13),
and
L-CDR1: RASESVEYYVTSLMQ (SEQ ID NO: 14)
L-CDR2: AASNVES (SEQ ID NO: 15)
L-CDR3: QQSRKVPYT (SEQ ID NO: 16), or
wherein the anti-CD28 antibody light and heavy chains comprise the following second combination of 6 CDRs :
as in Kabat:
H-CDR1: DYGVH (SEQ ID NO: 11)
H-CDR2: AIWAGGGTNYASSVMG (SEQ ID NO: 22)
H-CDR3: DKGYSYYYSMDY (SEQ ID NO: 13),
and
L-CDR1: RASESVEYYVTSLMA (SEQ ID NO: 23)
L-CDR2: AASNVES (SEQ ID NO: 15)
L-CDR3: QQSRKVPYT (SEQ ID NO: 16), or
wherein the anti-CD28 antibody light and heavy chains comprise the following third combination of 6 CDRs :
as in Kabat:
H-CDR1: GGSISSYY (SEQ ID NO:35)
H-CDR2: IYYSGIT (SEQ ID NO:36)
H-CDR3: ARWGVRRDYYYYGMDV (SEQ ID NO:37),
and
L-CDR1: QSVSSSY (SEQ ID NO:38)
L-CDR2: GAS (SEQ ID NO:39)
L-CDR3: QQYGSSPWT (SEQ ID NO:40), or
wherein the anti-CD28 antibody light and heavy chains comprise the following fourth combination of 6 CDRs :
as in Kabat:
H-CDR1 chosen from GFTFSSYG (SEQ ID NO:43) and GFTFSRNN (SEQ ID NO:44)
H-CDR2 chosen from ISYAGNNK (SEQ ID NO:45) and ISSNGGRT (SEQ ID NO:46)
H-CDR3 chosen from AKDSYYDFLTDPDVLDI (SEQ ID NO:47) and TRDDELLSFDY (SEQ ID NO:48),
and
L-CDR1: QSISSY (SEQ ID NO:49)
L-CDR2: AAS (SEQ ID NO:50)
L-CDR3: QQSYSTPPIT (SEQ ID NO:51), or
wherein the anti-CD28 antibody light and heavy chains comprise the following fifth combination of 6 CDRs :
as in Kabat:
H-CDR1: GFTFSRNN (SEQ ID NO:44)
H-CDR2: ISSNGGRT (SEQ ID NO:46)
H-CDR3: TRDDELLSFDY (SEQ ID NO:48),
and
L-CDR1: QSISSY (SEQ ID NO:49)
L-CDR2: AAS (SEQ ID NO:50)
L-CDR3: QQSYSTPPIT (SEQ ID NO:51), or
wherein the anti-CD28 antibody light and heavy chains comprise the following sixth combination of 6 CDRs :
as in Kabat:
H-CDR1: GYTFTSYY (SEQ ID NO:57)
H-CDR2: IYPGNVNT (SEQ ID NO:58)
H-CDR3: TRSHYGLDWNFDV (SEQ ID NO:59),
and
L-CDR1: QNIYVW (SEQ ID N0:60)
L-CDR2: KAS (SEQ ID NO:61)
L-CDR3: QQGQTYPY (SEQ ID NO:62), or
wherein the anti-CD28 antibody light and heavy chains comprise one of the following combinations of 6 CDRs :
as in Kabat:
H-CDR1: SYAMS (SEQ ID NO:65)
H-CDR2: TISGSGDSTYYADSVKG (SEQ ID NO:66)
H-CDR3: SGPGLRQVGFDY (SEQ ID NO:67)
L-CDR1 :RASQSISSYLN (SEQ ID NO:68)
L-CDR2: AASSLQS (SEQ ID NO:69)
L-CDR3: QQSYSTPFT (SEQ ID NO:70), or
H-CDR1: SYAMS (SEQ ID NO:65)
H-CDR2: TISGSGDSTYYADSVKG (SEQ ID NO:66)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQVYSTPFT (SEQ ID NO:71), or
H-CDR1: SYYMS (SEQ ID NO:72)
H-CDR2: TISGSGDSTYYADSVKG (SEQ ID NO:66)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQVYSTPFT (SEQ ID NO:71), or
H-CDR1: SYYMS (SEQ ID NO:72)
H-CDR2: TISESGDSTYYADSVKG (SEQ ID NO:73)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQSYSTPFT (SEQ ID NO:70), or
H-CDR1: SYYMS (SEQ ID NO:72)
H-CDR2: TISESGDSTYYADSVKG (SEQ ID NO:73)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQVYSTPFT (SEQ ID NO:71).

12. The bispecific protein complex of any one of claims 1 to 11, wherein the link between each C-terminal end of the CH3 domain of the heavy chain and the N-terminal end of a soluble PD-1 protein is made by a linker or short peptide fragment, preferably GGGGS, GGGSG, GGSGG, GSGGG or SGGGG, and more preferably the linker is (GGGGS)ₚ, with p being an integer from 1 to 5, preferably from 2 to 4, preferably 3.

13. The bispecific protein complex of any one of claims 1 to 12, wherein one of combinations a) to t) is fulfilled :
a)
the anti-CD28 VL domain is of sequence SEQ ID NO :1, and
the anti-CD28 VH domain is of sequence SEQ ID NO :2, and
the soluble PD-1 protein fragment is of sequence SEQ ID NO :3 ;
or
b)
the anti-CD28 VL domain is of sequence SEQ ID NO :1, and
the anti-CD28 VH domain is of sequence SEQ ID NO :2, and
the soluble PD-1 protein fragment is of sequence SEQ ID NO :4;
or
c) the anti-CD28 VH domain and the anti-CD28 VL domain comprise the following first combinations of 6 CDRs :
as in Kabat:
H-CDR1: DYGVH (SEQ ID NO: 11)
H-CDR2: VIWAGGGTNYNSALMS (SEQ ID NO: 12)
H-CDR3: DKGYSYYYSMDY (SEQ ID NO: 13),
and
L-CDR1: RASESVEYYVTSLMQ (SEQ ID NO: 14)
L-CDR2: AASNVES (SEQ ID NO: 15)
L-CDR3: QQSRKVPYT (SEQ ID NO: 16), and
the soluble PD-1 protein fragment is of sequence SEQ ID NO :3 ;
or
d) the anti-CD28 VH domain and the anti-CD28 VL domain comprise the following first combinations of 6 CDRs :
as in Kabat:
H-CDR1: DYGVH (SEQ ID NO: 11)
H-CDR2: VIWAGGGTNYNSALMS (SEQ ID NO: 12)
H-CDR3: DKGYSYYYSMDY (SEQ ID NO: 13),
and
L-CDR1: RASESVEYYVTSLMQ (SEQ ID NO: 14)
L-CDR2: AASNVES (SEQ ID NO: 15)
L-CDR3: QQSRKVPYT (SEQ ID NO: 16), and
the soluble PD-1 protein fragment is of sequence SEQ ID NO :4;
or
e) the anti-CD28 VH domain and the anti-CD28 VL domain comprise the following second combinations of 6 CDRs :
as in Kabat:
H-CDR1: DYGVH (SEQ ID NO: 11)
H-CDR2: AIWAGGGTNYASSVMG (SEQ ID NO: 22)
H-CDR3: DKGYSYYYSMDY (SEQ ID NO: 13),
and
L-CDR1: RASESVEYYVTSLMA (SEQ ID NO: 23)
L-CDR2: AASNVES (SEQ ID NO: 15)
L-CDR3: QQSRKVPYT (SEQ ID NO: 16), and
the soluble PD-1 protein fragment is of sequence SEQ ID NO :3 ;
or
f) the anti-CD28 VH domain and the anti-CD28 VL domain comprise the following second combinations of 6 CDRs :
as in Kabat:
H-CDR1: DYGVH (SEQ ID NO: 11)
H-CDR2: AIWAGGGTNYASSVMG (SEQ ID NO: 22)
H-CDR3: DKGYSYYYSMDY (SEQ ID NO: 13),
and
L-CDR1: RASESVEYYVTSLMA (SEQ ID NO: 23)
L-CDR2: AASNVES (SEQ ID NO: 15)
L-CDR3: QQSRKVPYT (SEQ ID NO: 16), and
the soluble PD-1 protein fragment is of sequence SEQ ID NO :4;
or
g) the anti-CD28 VH domain and the anti-CD28 VL domain are respectively of sequences SEQ ID NO:26 and SEQ ID NO:25, and the soluble PD-1 protein fragment is of sequence SEQ ID NO :3 ;
or
h) the anti-CD28 VH domain and the anti-CD28 VL domain are respectively of sequences SEQ ID NO:26 and SEQ ID NO:25, and the soluble PD-1 protein fragment is of sequence SEQ ID NO :4;
or
i) the anti-CD28 VH domain and the anti-CD28 VL domain comprise the following third combinations of 6 CDRs :
as in Kabat:
H-CDR1: GGSISSYY (SEQ ID NO:35)
H-CDR2: IYYSGIT (SEQ ID NO:36)
H-CDR3: ARWGVRRDYYYYGMDV (SEQ ID NO:37),
and
L-CDR1: QSVSSSY (SEQ ID NO:38)
L-CDR2: GAS (SEQ ID NO:39)
L-CDR3: QQYGSSPWT (SEQ ID NO:40), and
the soluble PD-1 protein fragment is of sequence SEQ ID NO :3 or 4;
or
j) the anti-CD28 VH domain and the anti-CD28 VL domain are respectively of sequences SEQ ID NO:42 and SEQ ID NO:41, and the soluble PD-1 protein fragment is of sequence SEQ ID NO :3 or 4;
k) the anti-CD28 VH domain and the anti-CD28 VL domain comprise the following fourth combinations of 6 CDRs :
as in Kabat:
H-CDR1 chosen from GFTFSSYG (SEQ ID NO:43) and GFTFSRNN (SEQ ID NO:44)
H-CDR2 chosen from ISYAGNNK (SEQ ID NO:45) and ISSNGGRT (SEQ ID NO:46)
H-CDR3 chosen from AKDSYYDFLTDPDVLDI (SEQ ID NO:47) and TRDDELLSFDY (SEQ ID NO:48),
and
L-CDR1: QSISSY (SEQ ID NO:49)
L-CDR2: AAS (SEQ ID NO:50)
L-CDR3: QQSYSTPPIT (SEQ ID NO:51), and
the soluble PD-1 protein fragment is of sequence SEQ ID NO :3 or 4;
or
l) the anti-CD28 VH domain and the anti-CD28 VL domain comprise the following fifth combinations of 6 CDRs :
as in Kabat:
H-CDR1: GFTFSRNN (SEQ ID NO:44)
H-CDR2: ISSNGGRT (SEQ ID NO:46)
H-CDR3: TRDDELLSFDY (SEQ ID NO:48),
and
L-CDR1: QSISSY (SEQ ID NO:49)
L-CDR2: AAS (SEQ ID NO:50)
L-CDR3: QQSYSTPPIT (SEQ ID NO:51), and
the soluble PD-1 protein fragment is of sequence SEQ ID NO :3 or 4;
or
m) the anti-CD28 VH domain and the anti-CD28 VL domain are respectively of sequences SEQ ID NO:56 and SEQ ID NO:55, and the soluble PD-1 protein fragment is of sequence SEQ ID NO :3 or 4;
or
n) the anti-CD28 VH domain and the anti-CD28 VL domain comprise the following sixth combinations of 6 CDRs :
as in Kabat:
H-CDR1: GYTFTSYY (SEQ ID NO:57)
H-CDR2: IYPGNVNT (SEQ ID NO:58)
H-CDR3: TRSHYGLDWNFDV (SEQ ID NO:59),
and
L-CDR1: QNIYVW (SEQ ID NO:60)
L-CDR2: KAS (SEQ ID NO:61)
L-CDR3: QQGQTYPY (SEQ ID NO:62), and
the soluble PD-1 protein fragment is of sequence SEQ ID NO :3 or 4;
or
o) the anti-CD28 VH domain and the anti-CD28 VL domain are respectively of sequences SEQ ID NO:64 and SEQ ID NO:63, and the soluble PD-1 protein fragment is of sequence SEQ ID NO :3 or 4;
p) the anti-CD28 VH domain and the anti-CD28 VL domain comprise the following seventh combinations of 6 CDRs :
as in Kabat:
H-CDR1: SYAMS (SEQ ID NO:65)
H-CDR2: TISGSGDSTYYADSVKG (SEQ ID NO:66)
H-CDR3: SGPGLRQVGFDY (SEQ ID NO:67)
L-CDR1 :RASQSISSYLN (SEQ ID NO:68)
L-CDR2: AASSLQS (SEQ ID NO:69)
L-CDR3: QQSYSTPFT (SEQ ID NO:70), and
the soluble PD-1 protein fragment is of sequence SEQ ID NO :3 or 4;
or
q) the anti-CD28 VH domain and the anti-CD28 VL domain comprise the following seventh combinations of 6 CDRs :
as in Kabat:
H-CDR1: SYAMS (SEQ ID NO:65)
H-CDR2: TISGSGDSTYYADSVKG (SEQ ID NO:66)
H-CDR3: SGPGLRQVGFDY (SEQ ID NO:67)
L-CDR1 :RASQSISSYLN (SEQ ID NO:68)
L-CDR2: AASSLQS (SEQ ID NO:69)
L-CDR3: QQVYSTPFT (SEQ ID NO:71), and
the soluble PD-1 protein fragment is of sequence SEQ ID NO :3 or 4;
or
r) the anti-CD28 VH domain and the anti-CD28 VL domain comprise the following seventh combinations of 6 CDRs :
as in Kabat:
H-CDR1: SYYMS (SEQ ID NO:72)
H-CDR2: TISGSGDSTYYADSVKG (SEQ ID NO:66)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQVYSTPFT (SEQ ID NO:71), and
the soluble PD-1 protein fragment is of sequence SEQ ID NO :3 or 4;
or
s) the anti-CD28 VH domain and the anti-CD28 VL domain comprise the following seventh combinations of 6 CDRs :
as in Kabat:
H-CDR1: SYYMS (SEQ ID NO:72)
H-CDR2: TISESGDSTYYADSVKG (SEQ ID NO:73)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQSYSTPFT (SEQ ID NO:70), and
the soluble PD-1 protein fragment is of sequence SEQ ID NO :3 or 4;
or
t) the anti-CD28 VH domain and the anti-CD28 VL domain comprise the following seventh combinations of 6 CDRs :
as in Kabat:
H-CDR1: SYYMS (SEQ ID NO:72)
H-CDR2: TISESGDSTYYADSVKG (SEQ ID NO:73)
H-CDR3: SGPGLRQVGFDY (SEQ ID NO:67)
L-CDR1:RASQSISSYLN (SEQ ID NO:68)
L-CDR2: AASSLQS (SEQ ID NO:69)
L-CDR3: QQVYSTPFT (SEQ ID NO:71), and
the soluble PD-1 protein fragment is of sequence SEQ ID NO :3 or 4.

14. The bispecific protein complex of any one of claims 1 to 13, for use as a medicament.

15. The bispecific protein complex of any one of claims 1 to 13, for use for treating a cancer.
